# EUROPEAN PATENT APPLICATION

(11) **EP 4 696 714 A1**
(43) Date of publication of application: **18.02.2026**
(21) Application number: 24787854.9
(22) Date of filing: 08.03.2024
(51) Int. Cl.: C07K 16/36, C07K 7/02, A61P 35/00, C07K 16/46, C07K 19/00, C12N 5/10, A61K 39/395, A61K 47/68, A61P 35/04, G01N 33/86, G01N 33/574, G01N 33/53

(54) **PREPARATION METHOD FOR NANOBODY TARGETING TISSUE FACTOR AND CONJUGATE AND USE THEREOF**

(30) Priority: 10.04.2023 CN 202310375192; 04.05.2023 WO PCT/CN2023/092105
(71) Applicant: Fudan University, Shanghai 200433 (CN)
(72) Inventor: YU, Ke, Shanghai 200433 (CN); JIN, Rui, Shanghai 200433 (CN); WANG, Yue, Shanghai 200433 (CN); LIU, Liang, Shanghai 200433 (CN)
(74) Representative: Mewburn Ellis LLP
(86) International application number: PCT/CN2024/080820
(87) International publication number: WO 2024/212748

(57) **Abstract**

Disclosed are a new nanobody (Nb) targeting a tissue factor (TF) and a nanobody-drug conjugate (NDC), a preparation method therefor and the use thereof. The monoclonal nanobody and the corresponding NDC can efficiently and high-specifically bind to a purified TF protein and a TF on the surface of various TF abnormally expressed tumor cells, have a high affinity and a low immunogenicity, and have a significant anti-tumor effect in vivo and in vitro.

## Description

### TECHNICAL FIELD

The present invention relates to the field of medicine, in particular to a nanobody targeting tissue factor and a nanobody-drug conjugate, a preparation method therefor, and use thereof.

### BACKGROUND

Tissue factor (TF) is a transmembrane glycoprotein with a molecular weight of 47 kDa. Under physiological conditions, TF activates the extrinsic coagulation reaction upon vascular injury. However, expression of TF is abnormally activated in many tumor tissues, exhibiting high abnormal expression rates in breast cancer, pancreatic cancer, lung cancer, and esophageal cancer, etc. For example, the abnormal expression rate of TF is 85.8% in breast cancer, 88.5% in pancreatic cancer, 83.6% in lung cancer, 91.3% in esophageal cancer, etc. (Blood, 2012, 119: 924-932). The TF/FVIIa complex can directly bind to transmembrane G protein coupled receptor Protease-activated receptor 2 (PAR2) and induce its activation. TF can affect a series of tumor functional signaling within cells through PAR2. For example, TF-PAR2 induces gene expression of key growth factors, immune regulatory factors, and chemokines through MAPK/ERK phosphorylation, promoting angiogenesis and providing sufficient nutrients, energy, and a suitable microenvironment for tumor growth. In addition, TF can promote the migration and adhesion of tumor cells by interacting with Rac1 and β1 families related integrins, thereby enhancing the overall hematogenous metastasis ability of tumor cells (Journal of Thrombosis Research, 2012, 130: S84-S87; Journal of Thrombosis and Haemostasis, 2013, 11:285-293; International Journal of Cancer, 2014, doi: 10.1002/ijc.28959; Blood, 2012, 119: 924-932). Therefore, given the current international research on the role and function of TF related diseases, developing specific therapeutic antibodies targeting TF is extremely beneficial for the diagnosis, treatment, and prevention of pathological features of vascular proliferation, abnormal coagulation, and the like caused by TF in various diseases such as cancer, thrombosis, and inflammation. The world's first anti-tumor drug targeting TF, Tivdak (TF-ADC Tisotumab Vedotin), has been approved for market in 2021, and several TF-ADCs developed by different pharmaceutical companies are in the clinical research stage.

Monoclonal antibody drug has brought breakthroughs in cancer treatment and improvement of clinical treatments in multiple cancer fields. However, the molecular weight of traditional monoclonal antibody is 150 kd, which limits its penetration and drug distribution in solid tumors, and the therapeutic effect still needs to be further improved. Nanobody (Nb) is an antibody naturally produced by alpacas, consisting only of heavy chains. The target recognition module is consisted of a single heavy chain variable region (VHH) with a molecular weight of only 12-14 kd, which is expected to overcome the low penetration of traditional monoclonal antibody into solid tumors. Nanobodies can be linked to functional domains such as Fc, other nanobodies, peptide tags or toxins. Due to its small size, nanobody demonstrates higher diffusion rate, and strong vascular permeability, penetration of blood-brain and other *in vivo* barriers, and tumor penetration, enabling more uniform tissue distribution than traditional monoclonal antibody. These characteristics make them particularly suitable for specific and effective tumor targeted therapy *in vivo.* Nanobodies also have advantages such as low immunogenicity, high stability, high degradation resistance, low production cost of prokaryotic expression, and easy product characterization. However, despite numerous potential advantages, the development of tumor therapy drugs based on nanobodies is still in the early stage of exploration and research.

In recent years, antibody-drug conjugate (ADC) has become a new trend in tumor therapy. ADC utilizes monoclonal antibodies to specifically recognize specific antigens on the surface of tumor cells, so as to accurately deliver and release anti-tumor drugs (such as small molecule chemotherapy drugs) to tumor target cells, to achieve the purpose of precise tumor killing. However, due to the limitations of large molecular weight and weak intratumoral penetration of ADC, the effect of drug absorption and distribution in tumors is limited and has large difference, and the application in solid tumors is not ideal. Moreover, ADC has a long terminal half-life in the circulatory system *in vivo,* which can lead to unnecessary toxicity of the loaded toxin to normal tissues or cells. The nanobody drug conjugate (NDC) prepared by nanobody not only retains the advantages of traditional ADC, but also has high vascular permeability, good blood-brain barrier penetration, strong tumor penetration, and fast speed of reaching target cells. It can improve the accumulation of drugs in tumors, and also moderately control the plasma exposure and half-life of drugs, which can further improve the therapeutic effect and overall therapeutic window for solid tumors. It is expected to become the most potential class of new anti-tumor drugs.

In summary, based on the important role of TF in tumor development, relapse and drug resistance, the development of targeted TF-Nb and TF-NDC drugs can provide new strategies of single or combined drug therapy for the clinical treatment of patients with abnormal expression of TF.

### SUMMARY OF THE INVENTION

The object of the present invention is to provide a nanobody targeting TF and a nanobody-drug conjugate, a preparation method therefor and use thereof.

In the first aspect of the present invention, it provides a nanobody targeting TF, wherein the complementary determining region (CDR) of the VHH chain of the nanobody is one or more selected from the following groups:
(1) CDR1 as shown in SEQ ID NO.1,
   CDR2 as shown in SEQ ID NO.2, and
   CDR3 as shown in SEQ ID NO.3;
   or,
(2) CDR1 as shown in SEQ ID NO.5,
   CDR2 as shown in SEQ ID NO.6, and
   CDR3 as shown in SEQ ID NO.7;
   or,
(3) CDR1 as shown in SEQ ID NO.9,
   CDR2 as shown in SEQ ID NO.10, and
   CDR3 as shown in SEQ ID NO.11,
   or,
(4) CDR1 as shown in SEQ ID NO.13,
   CDR2 as shown in SEQ ID NO.14, and
   CDR3 as shown in SEQ ID NO.15,
   or,
(5) CDR1 as shown in SEQ ID NO.17,
   CDR2 as shown in SEQ ID NO.18, and
   CDR3 as shown in SEQ ID NO.19,
   or,
(6) CDR1 as shown in SEQ ID NO.21,
   CDR2 as shown in SEQ ID NO.22, and
   CDR3 as shown in SEQ ID NO.23,
   or,
(7) CDR1 as shown in SEQ ID NO.25,
   CDR2 as shown in SEQ ID NO.26, and
   CDR3 as shown in SEQ ID NO.27;
or, in another preferred embodiment, any one of the above amino acid sequences further comprises a derivative sequence that is optionally added, deleted, modified and/or substituted with at least one amino acid and may retain the binding affinity to TF.

In another preferred embodiment, the CDR region of the VHH chain of the nanobody comprises an amino acid sequence having at least 80%, preferably at least 90%, more preferably at least 95%, and even more preferably at least 99% sequence identity to any one of aforementioned CDR region sequences.

In another preferred embodiment, the amino acid sequence of the CDR region of VHH chain of the nanobody comprises one or more amino acid substitutions, preferably conservative amino acid substitutions, compared with any one of aforementioned CDR region sequences.

In another preferred embodiment, the VHH chain comprises CDR1, CDR2 and CDR3 selected from the group consisting of:
(1) the complementary determining regions CDR1, CDR2, and CDR3 as shown in SEQ ID NO.1, SEQ ID NO.2, and SEQ ID NO.3 (corresponding to the CDRs of nanobody 4-A02); or
(2) the complementary determining regions CDR1, CDR2, and CDR3 as shown in SEQ ID NO.5, SEQ ID NO.6, and SEQ ID NO.7 (corresponding to the CDRs of nanobody F1-A01); or
(3) the complementary determining regions CDR1, CDR2, and CDR3 as shown in SEQ ID NO.9, SEQ ID NO.10, and SEQ ID NO.11 (corresponding to the CDRs of nanobody 4-C08); or
(4) the complementary determining regions CDR1, CDR2, and CDR3 as shown in SEQ ID NO.13, SEQ ID NO.14, and SEQ ID NO.15 (corresponding to the CDRs of nanobody 4-E04); or
(5) the complementary determining regions CDR1, CDR2, and CDR3 as shown in SEQ ID NO.17, SEQ ID NO.18, and SEQ ID NO.19 (corresponding to the CDRs of nanobody 4-C05); or
(6) the complementary determining regions CDR1, CDR2, and CDR3 as shown in SEQ ID NO.21, SEQ ID NO.22, and SEQ ID NO.23 (corresponding to the CDRs of nanobody 5-C12); or
(7) the complementary determining regions CDR1, CDR2, and CDR3 as shown in SEQ ID NO.25, SEQ ID NO.26, and SEQ ID NO.27 (corresponding to the CDRs of nanobody 5-H3),

In another preferred embodiment, the CDR1, CDR2, and CDR3 are separated by the framework regions FR1, FR2, FR3, and FR4 of the VHH chain.

In another preferred embodiment, the nanobody comprises a humanized antibody, a camel-derived antibody, a chimeric antibody.

In another preferred embodiment, the VHH chain of the nanobody further comprises a framework region (FR).

In another preferred embodiment, the framework region (FR) is derived from human, murine, rabbit or camel.

In another preferred embodiment, the framework region (FR) comprises a human-derived FR region, a murine-derived FR region or a camel-derived FR region.

In another preferred embodiment, the VHH chain of the nanobody targeting TF has an amino acid sequence as shown in SEQ ID NO.4, SEQ ID NO.8, SEQ ID NO.12, SEQ ID NO.16, SEQ ID NO.20, SEQ ID NO.24, or SEQ ID NO.28.

In another preferred embodiment, the VHH chain of the nanobody targeting TF has an amino acid sequence as shown in positions 1-114 of SEQ ID NO.33, positions 1-114 of SEQ ID NO.34, or positions 1-114 of SEQ ID NO.35.

In another preferred embodiment, the sequence of the nanobody comprises an amino acid sequence having at least 80%, preferably at least 90%, more preferably at least 95%, and even more preferably at least 99% sequence identity to SEQ ID NO.4, SEQ ID NO.8, SEQ ID NO.12, SEQ ID NO.16, SEQ ID NO.20, SEQ ID NO.24, or SEQ ID NO.28.

In the second aspect of the present invention, it provides an antibody targeting TF, which comprises one or more VHH chains of the nanobody targeting TF of the first aspect of the present invention.

In another preferred embodiment, the VHH chain of the nanobody targeting TF has an amino acid sequence selected from the group consisting of SEQ ID NO.4, SEQ ID NO.8, SEQ ID NO.12, SEQ ID NO.16, SEQ ID NO.20, SEQ ID NO.24, and SEQ ID NO.28.

In another preferred embodiment, the antibody is a monomer, a bivalent antibody, and/or a multivalent antibody.

In another preferred embodiment, the antibody is an animal derived antibody, a humanized antibody, a chimeric antibody or a chimeric antigen receptor antibody (CAR).

In another preferred embodiment, the CDR regions of the humanized antibody comprise 1, 2, or 3 amino acid changes.

In another preferred embodiment, the animal is a non-human mammal, preferably a mouse, sheep, rabbit or camel.

In another preferred embodiment, the antibody is a double chain antibody or a single chain antibody.

In another preferred embodiment, the antibody is a monoclonal antibody.

In another preferred embodiment, the antibody is a partially or fully humanized monoclonal antibody.

In another preferred embodiment, the antibody is a humanized antibody, and the VHH chain of the nanobody targeting TF has an amino acid sequence as shown in positions 1-114 of SEQ ID NO.33, positions 1-114 of SEQ ID NO.34, or positions 1-114 of SEQ ID NO.35.

In another preferred embodiment, the number of added, deleted, modified and/or substituted amino acids does not exceed 40%, preferably 20%, and more preferably 10% of the total number of amino acids in the original amino acid sequence.

In another preferred embodiment, the number of added, deleted, modified and/or substituted amino acids is 1-7, preferably 1-3, and more preferably 1.

In another preferred embodiment, the amino acid sequence that is added, deleted, modified and/or substituted with at least one amino acid is an amino acid sequence having a homology of at least 80%.

In another preferred embodiment, the derivative sequence that is added, deleted, modified and/or substituted with at least one amino acid has the function of inhibiting TF on cell surface or recombinant TF protein.

In another preferred embodiment, the antibody is in the form of a drug conjugate.

In another preferred embodiment, the binding constant KD value of the nanobody to extracellular domain of human TF protein (TF-EDC) measured by ForteBio surface plasmon resonance (SPR) ranges from 0.202~7.04nM.

In another preferred embodiment, the antibody has one or more properties selected from the group consisting of:
(a) specifically binding to TF on tumor cells and/or immune/stromal cells in the tumor microenvironment;
(b) specifically inhibiting TF-associated tumor cell signaling pathways;
(c) improving disorder of cytokine secretion caused by TF overactivation;
(d) inhibiting migration or metastasis of tumor cell;
(e) suppressing tumor growth and enhancing the anti-tumor efficacy of combination therapy;
(f) promoting proliferation, survival, and function of immune cell, thereby improving the effect of tumor immunotherapy;
(g) being internalized into intracellular lysosomes after binding to tumor cells;
(h) exhibiting blood-brain barrier penetration and efficient brain distribution;
(i) demonstrating good therapeutic efficacy against brain tumors;
(j) demonstrating good therapeutic efficacy against multiple solid tumors.

In the third aspect of the present invention, it provides a multispecific antibody, wherein the multispecific antibody comprises: the nanobody targeting TF of the first aspect of the present invention or the antibody targeting TF of the second aspect of the present invention.

In another preferred embodiment, the multispecific antibody further comprises a second antigen binding region targeting a target selected from the group consisting of: EGFR, TGFβ, BCMA, B7H6, GUCY2C, DLL3, CD38, CD123, CD19, CD20, CD22, B7-H3, GPC3, HER2, PMSA, CD28, 4-1BB, OX40, CD40, CD27, CD3, CTLA4, PD1, PDL1, CD73, BCMA, GLP-1, Trop2, TIGIT, LAG-3, FGL1, TLR7, and a combination thereof.

In another preferred embodiment, the second antigen binding region is a nanobody.

In another preferred embodiment, the multispecific antibody comprises one or more second antigen binding regions.

In another preferred embodiment, the multispecific antibody further comprises an antibody Fc segment.

In another preferred embodiment, the antigen binding region is an antibody or an antibody fragment, wherein the antibody fragment comprises: (i) Fab fragment; (ii) F(ab')₂ fragment; (iii) Fd fragment; (iv) Fv fragment; (v) single-chain Fv (scFv) molecule; (vi) dAb fragment.

In the fourth aspect of the present invention, it provides a recombinant protein, wherein the recombinant protein comprises:
(i) the nanobody targeting TF of the first aspect of the invention, the antibody targeting TF of the second aspect of the invention, or the multispecific antibody of the third aspect of the invention; and
(ii) an optional polypeptide molecule or fragment with therapeutic function; and/or
(iii) an optional functional domain for improvement of the physicochemical properties or druggability of the protein.

In another preferred embodiment, the improvement of the physicochemical properties or drugability of the protein includes prolonging the half-life of the nanobody targeting TF.

In another preferred embodiment, the recombinant protein further comprises: (iv) an optional tag sequence for facilitating expression and/or purification.

In another preferred embodiment, the tag sequence is selected from the group consisting of: 6His tag, GGGS sequence, FLAG tag.

In another preferred embodiment, the recombinant protein is a monomer, dimer, or polymer.

In another preferred embodiment, the polypeptide molecule or fragment with therapeutic function includes but is not limited to: polypeptide molecule or fragment targeting EGFR, TGFβ, BCMA, B7H6, GUCY2C, DLL3, CD38, CD123, CD19, CD20, CD22, B7-H3, GPC3, HER2, PMSA, CD28, 4-1BB, OX40, CD40, CD27, CD3, CTLA4, PD1, PDL1, CD73, BCMA, GLP-1, Trop2, TIGIT, LAG-3, FGL1, TLR7.

In another preferred embodiment, the polypeptide molecule or fragment with therapeutic function includes but is not limited to: insulin, IL-2, interferon, calcitonin, GHRH peptide, intestinal peptide analog, albumin, antibody fragment, and cytokine.

In another preferred embodiment, the recombinant protein (or polypeptide) comprises a fusion protein.

In another preferred embodiment, the fusion protein comprises a multispecific antibody, and a chimeric antibody.

In another preferred embodiment, the functional domain for improvement of the physicochemical properties or druggability of a protein includes an Fc segment, an anti-albumin nanobody (HLE), and an albumin binding domain (ABD).

In another preferred embodiment, the recombinant protein has the following structure from the N to C terminus or from the C to N terminus:

(A-B)m;

wherein the element A is the nanobody targeting TF;
the element B is an Fc segment, an albumin binding domain (ABD) or an anti-albumin nanobody (HLE);
"-" represents a peptide bond or linker,
wherein m is a positive integer.

In another preferred embodiment, the linker is a rigid linker or a flexible linker.

In another preferred embodiment, the element B further comprises an optional functional domain for improvement of the physicochemical properties or druggability of the protein, or an optional tag sequence for facilitating expression and/or purification.

In another preferred embodiment, m is 1, 2, 3 or 4.

In another preferred embodiment, the nanobody targeting TF is the nanobody targeting TF of the first aspect of the invention, the antibody targeting TF of the second aspect of the invention, or the multispecific antibody of the third aspect of the invention.

In another preferred embodiment, the VHH chain of the nanobody targeting TF has an amino acid sequence selected from the group consisting of: SEQ ID NO.4, SEQ ID NO.8, SEQ ID NO.12, SEQ ID NO.16, SEQ ID NO.20, SEQ ID NO.24, and SEQ ID NO.28.

In another preferred embodiment, the Fc segment is a human IgG Fc segment, preferably it is derived from human IgG1 or human IgG2.

In another preferred embodiment, the Fc segment is selected from FC1, FCWT and FCLALAPG; wherein
the sequence of the FC1 is as shown in positions 115-346 of SEQ ID NO.29;
the sequence of the FCWT is as shown in positions 115-346 of SEQ ID NO.30;
the sequence of the FCLALAPG is as shown in positions 115-346 of SEQ ID NO.31.

In another preferred embodiment, the amino sequence of ABD is as shown in positions 130-198 of SEQ ID NO.32.

In another preferred embodiment, the recombinant protein has an amino sequence selected from the group consisting of: SEQ ID NO.29, SEQ ID NO.30, SEQ ID NO.31, and SEQ ID NO.32.

In another preferred embodiment, the recombinant protein comprises a humanized antibody, preferably has an amino sequence selected from the group consisting of: positions 1-114 of SEQ ID NO.33, positions 1-114 of SEQ ID NO.34, and positions 1-114 of SEQ ID NO.35.

In the fifth aspect of the present invention, it provides a CAR construct, wherein the antigen binding region of the CAR construct is the VHH chain of the nanobody of the first aspect of the present invention.

In the sixth aspect of the present invention, it provides a recombinant immune cell, wherein the immune cell expresses the exogenous CAR construct of the fifth aspect of the present invention.

In another preferred embodiment, the immune cell is selected from the group consisting of: a NK cell and a T cell.

In another preferred embodiment, the immune cell is derived from human or non-human mammal (such as mouse).

In the seventh aspect of the present invention, it provides an immunoconjugate, wherein the immunoconjugate comprises:
(a) an antibody moiety, wherein the antibody moiety is the nanobody targeting TF of the first aspect of the invention or the antibody targeting TF of the second aspect of the invention; and
(b) a coupling moiety coupled to the nanobody moiety, wherein the coupling moiety is selected from the group consisting of: a detectable label, a drug, a toxin, a cytokine, an enzyme, a protein degradation agent, an immune regulator (including immune agonist and inhibitor), an oligonucleotide, and a combination thereof.

In another preferred embodiment, the immunoconjugate is a nanobody-drug conjugate (NDC).

In another preferred embodiment, the nanobody moiety is coupled to the coupling moiety via a chemical bond or a linker.

In another preferred embodiment, the coupling moiety is a chemical label or a biological label.

In another preferred embodiment, the chemical label is an isotope, an immunotoxin and/or a chemical drug.

In another preferred embodiment, the biological label is a biotin, an avidin or an enzyme marker.

In another preferred embodiment, the coupling moiety is a drug or a toxin.

In another preferred embodiment, the drug is a cytotoxic drug.

In another preferred embodiment, the cytotoxic drug is selected from the group consisting of: an antitubulin drug, DNA sulcus binding reagent, DNA replication inhibitor, alkylation reagent, antibiotic, folic acid antagonist, antimetabolite, chemosensitizer, topoisomerase inhibitor, vinca alkaloids, and a combination thereof.

Particularly useful cytotoxic drug includes, for example, DNA sulcus binding reagent, DNA alkylation reagent, and tubulin inhibitor. The typical cytotoxic drug includes, such as auristatins, camptothecins, duocarmycins, etoposides, maytansines and maytansinoids (such as DM1 and DM4), taxanes, benzodiazepines, or benzodiazepines containing drugs (for example pyrrolo [1,4] benzodiazepine (PBD), indolinobenzodiazepines, and oxazolidinobenzodiazepines, vinca alkaloids, and a combination thereof.

In another preferred embodiment, the toxin is selected from the group consisting of:
auristatin (e.g., auristatin E, auristatin F, MMAE and MMAF), chlortetracycline, maytansinoid, ricin, ricin A-chain, combretastatin, duocarmycin, dolastatin, adriamycin, daunorubicin, paclitaxel, cisplatin, cc1065, ethidium bromide, mitomycin, etoposide, tenoposide, vincristine, vinblastine, colchicine, dihydroxy anthrax dione, actinomycin, diphtheria toxin, pseudomonas exotoxin (PE)A, PE40, abrin, abrin A-chain, modeccin A-chain, α-octococcus, gelonin, mitogellin, retstrictocin, phenomycin, enomycin, curicin, crotonin, calicheamicin, Sapaonaria officinalis inhibitors, glucocorticoids, and a combination thereof.

In another preferred embodiment, the coupling moiety is a detectable label.

In another preferred embodiment, the detectable label comprises a radionuclide, and the radionuclide comprises:
(i) a diagnostic isotope selected from the group consisting of: Tc-99m, Ga-68, F-18, 1-123, I-125, I-131, In-111, Ga-67, Cu-64, Zr-89, C-11, Lu-177, Re-188, and a combination thereof; and/or
(ii) a therapeutic isotope selected from the group consisting of: Lu-177, Y-90, Ac-225, As-211, Bi-212, Bi-213, Cs-137, Cr-51, Co-60, Dy-165, Er-169, Fm-255, Au-198, Ho-166, I-125, 1-131, Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133 Yb-169, Yb-177, and a combination thereof.

In another preferred embodiment, the protein degradation agent is a degradation agent of a tumor-related protein selected from the group consisting of: EGFR, NF-κB, RIPK2, BCR-ABL, HER2, c-Met, TBK1, CDK, ALK, Akt, CK2, ERK1/2, FLT3, PI3K, BTK, TRK, Fak, BRD, AR, ER, MetAp-2, BCL-XL, Sirt2, HDAC6, Pirin, SMAD3, ARNT, PCAF/GCN5, Tau, EZH2, IRAK4, STAT3FRS2, and RAS (such as KRAS, HRAS and NRAS).

In another preferred embodiment, the KRAS includes KRAS-G12C, KRAS-G12D, KRAS-G12V, etc.

In another preferred embodiment, the protein degradation agent includes a PROTAC (proteolysis targeting chimera).

In another preferred embodiment, the PROTAC targets EGFR, KRAS (including KRAS-G12C, KRAS-G12D, KRAS-G12V, etc.).

In another preferred embodiment, the oligonucleotide includes an antisense oligonucleotide (ASO), a small interfering RNA (siRNA), a micro RNA (miRNA), a small activating RNA (saRNA), a messenger RNA (mRNA) or a RNA aptamer.

In another preferred embodiment, the coupling moiety is selected from: a fluorescent or luminescent marker, a radioactive marker, MRI (magnetic resonance imaging) or CT (computed tomography) contrast agent, or an enzyme capable of producing detectable product, a radionuclide, a biological toxin, a cytokine (such as IL-2), an antibody, an antibody Fc fragment, an antibody scFv fragment, a gold nanoparticle/nanorod, a viral particle, a liposome, a nanomagnetic particle, a prodrug activating enzyme (such as DT-cardiomyolase (DTD) or biphenyl hydrolase-like protein (BPHL)), chemotherapeutic agent (e.g., cisplatin) or a nanoparticle in any form, etc.

In another preferred embodiment, the immunoconjugate comprises: a multivalent (such as bivalent) nanobody targeting TF of the first aspect of the present invention or antibody targeting TF of the second aspect of the present invention.

In another preferred embodiment, the "multivalent" means that the amino acid sequence of the immunoconjugate comprises multiple repeats of the nanobody targeting TF of the first aspect of the present invention or the antibody targeting TF of the second aspect of the present invention.

In another preferred embodiment, the detection is *in vivo* detection or *in vitro* detection.

In another preferred embodiment, the immunoconjugate is used for diagnosing and/or treating a tumor that expresses TF protein.

In another preferred embodiment, the antibody-drug conjugate ADC is as shown in the following molecular formula: 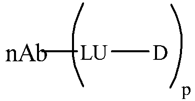 wherein:
nAb is the nanobody targeting TF or the recombinant protein of the fourth aspect of the present invention.
LU is linker 2 (also called a connector);
D is a drug;
and the subscript p is a value selected from 1-8.

In another preferred embodiment, nAb is the recombinant protein of the fourth aspect of the present invention.

In another preferred embodiment, the antibody-drug conjugate (ADC) is a monomer, dimer, or polymer.

In another preferred embodiment, the recombinant protein has the following structure from the N to C terminus or from the C to N terminus:

(A-L1-B)m;

wherein the element A is the nanobody targeting TF;
the element B is an Fc segment, an albumin binding domain (ABD) or an anti-albumin nanobody (HLE);
"L1" is absent or linker 1,
wherein m is a positive integer.

In another preferred embodiment, m is 1, 2, 3 or 4.

In another preferred embodiment, the drug is site-specifically conjugated to the terminal amino group, side-chain amino group, or sulfhydryl group of the nanobody targeting TF.

In another preferred embodiment, the drug is site-specifically or randomly conjugated to the nanobody targeting TF.

In another preferred embodiment, the drug is site-specifically conjugated to the element A, the element B, or L1.

In another preferred embodiment, the drug is site-specifically conjugated to the element B and/or L1.

In another preferred embodiment, the drug is site-specifically conjugated to the terminal amino group or sulfhydryl group of the element B; and/or the side-chain amino group or sulfhydryl group of L1.

In another preferred embodiment, the element B is an Fc segment, and the drug is site-specifically conjugated to the hinge region of the Fc segment; and/or the C-terminus of the Fc segment.

In another preferred embodiment, the element B is a human albumin-binding domain (ABD), and the drug is site-specifically conjugated to the C-terminus of the ABD.

In another preferred embodiment, the element B is HLE, and the drug is site-specifically conjugated to the C-terminus of HLE.

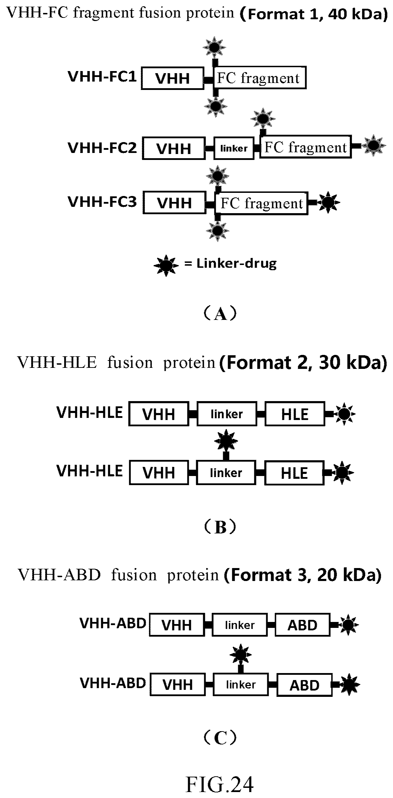

In another preferred embodiment, the antibody-drug conjugate (ADC) has a structure selected from those shown in Figure 24.

In another preferred embodiment, LU is a connector linked to the antibody moiety via a linker selected from the group consisting of maleimidocaproyl (MC), maleimide (MAL), and succinimidyl 4-(N-maleimidomethyl) cyclohexane-1-arboxylate) (SMCC), and comprises one or more selected from the group consisting of valine-citrulline (VC), valine-alanine (VA), glycine-glycine-phenylalanine-glycine (GGFG), alanine-alanine-alanine (AAA), p-aminobenzyloxycarbonyl (PAB), and polyethylene glycol (PEG).

In another preferred embodiment, the antibody is covalently bound to the linker by reacting with a moiety selected from the group consisting of maleimidyl hexanoyl (MC), maleimide (MAL), and succinimidyl 4-(N-maleimidomethyl)cyclohexane-1-carboxylate) (SMCC).

In another preferred embodiment, D is a compound with anti-tumor activity selected from the group consisting of:
(i) a tubulin inhibitor, such as maytansine derivative (DM1, DM4), monomethyl auristatin E (MMAE), and monomethyl auristatin F (MMAF);
(ii) a toxin that acts on DNA, such as duocarmycin and pyrrolobenzodiazepine (PBD);
(iii) a topoisomerase inhibitor, camptothecin, SN38, Exatecan, Dxd.

In another preferred embodiment, the compound LU-D is selected from the group consisting of:

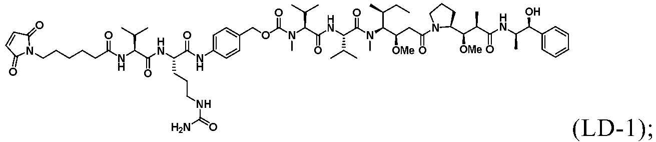

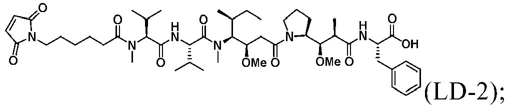

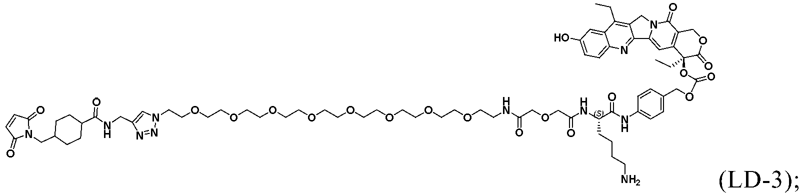

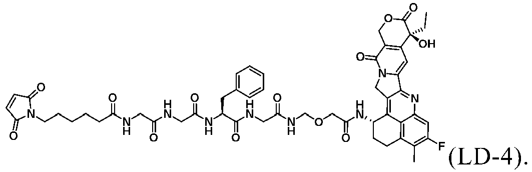

In the eighth aspect of the present invention, it provides a pharmaceutical composition comprising:
(i) the nanobody targeting TF of the first aspect of the present invention, the antibody targeting TF of the second aspect of the present invention, the multispecific antibody of the third aspect of the present invention, the recombinant protein of the fourth aspect of the present invention, the recombinant immune cell of the sixth aspect of the present invention, or the immunoconjugate of the seventh aspect of the present invention; and
(ii) a pharmaceutically acceptable carrier.

In another preferred embodiment, the pharmaceutical composition comprises a single drug, a compound drug, or a synergistic drug.

In another preferred embodiment, the pharmaceutical composition further comprises other biologically active substances, such as drugs for treating tumors.

In another preferred embodiment, the administration method of the pharmaceutical composition is selected from the group consisting of: subcutaneous injection, intradermal injection, intramuscular injection, intravenous injection, intraperitoneal injection, microneedle injection, oral administration, oral and nasal spraying, and aerosol inhalation.

In another preferred embodiment, the dosage form of the pharmaceutical composition is selected from the group consisting of: liquid, solid, and gel.

In another preferred embodiment, the pharmaceutical composition is a liquid formulation.

In another preferred embodiment, the pharmaceutical composition is an injection.

In the ninth aspect of the present invention, it provides a use of an active ingredient, wherein the active ingredient is selected from the group consisting of: the nanobody targeting TF of the first aspect of the present invention, the antibody targeting TF of the second aspect of the present invention, the multispecific antibody of the third aspect of the present invention, the recombinant protein of the fourth aspect of the present invention, the recombinant immune cell of the sixth aspect of the present invention, the immunoconjugate of the seventh aspect of the present invention, and a combination thereof, wherein the active ingredient is used for (a) preparing a detection reagent, a detection plate or a kit; and/or (b) preparing a medicine for the prevention and/or treatment of a TF-related disease.

In another preferred embodiment, the detection reagent, detection plate or kit is used in:
(1) detection of TF protein in a sample; and/or
(2) detection of endogenous TF protein in a tumor cell; and/or
(3) detection of a tumor cell expressing TF protein.

In another preferred embodiment, the type of the detection includes but is not limited to flow cytometry, cell immunofluorescence detection, enzyme-linked immunosorbent assay, immunoblotting detection, etc.

In another preferred embodiment, the detection reagent, detection plate or kit is used for diagnosing a TF-related disease.

In another preferred embodiment, the drug is used for treating or preventing a tumor, tumor migration, or tumor resistance with a high TF expression.

In another preferred embodiment, the tumor resistance includes: resistance to a tumor immunotherapy drug, resistance to a tumor targeted therapy drug, resistance to conventional tumor chemotherapy, and insensitivity to radiotherapy.

In another preferred embodiment, the drug is used for a purpose selected from the group consisting of:
(a) specifically binding to TF on tumor cells and/or immune/stromal cells in the tumor microenvironment;
(b) specifically inhibiting TF-associated tumor cell signaling pathways;
(c) improving disorder of cytokine secretion caused by TF overactivation;
(d) inhibiting migration or metastasis of tumor cell;
(e) suppressing tumor growth and enhancing the anti-tumor efficacy of combination therapy;
(f) promoting proliferation, survival, and function of immune cell, thereby improving the effect of tumor immunotherapy;
(g) being internalized into intracellular lysosomes after binding to tumor cells;
(h) exhibiting blood-brain barrier penetration and efficient brain distribution;
(i) demonstrating good therapeutic efficacy against brain tumors;
(j) demonstrating good therapeutic efficacy against multiple solid tumors.

In another preferred embodiment, the TF-related disease is selected from the group consisting of: a cancer, thrombosis, autoimmune disease, metabolic-related disease, infectious disease, and a combination thereof.

In another preferred embodiment, the TF-related disease comprises: tumor occurrence, growth and/or metastasis.

In another preferred embodiment, the cancer comprises a solid tumor and a hematologic cancer.

In another preferred embodiment, the cancer is a tumor with a high TF expression.

In another preferred embodiment, the tumor with a high TF expression is selected from the group consisting of: breast cancer, lung cancer, pancreatic cancer, ovarian cancer, prostate cancer, colorectal cancer, glioma, melanoma, leukemia, lymphoma, and a combination thereof.

In another preferred embodiment, the cancer is a drug-resistant tumor.

In another preferred embodiment, the tumor with a high TF expression refers to the ratio of the level L1 of TF transcript and/or protein in a tumor tissue to the level L0 of TF transcript and/or protein in a normal tissue, and L1/L0 is ≥ 2, preferably ≥ 3.

In the tenth aspect of the present invention, it provides a polynucleotide encoding a polypeptide selected from group consisting of:
(1) the nanobody targeting TF of the first aspect of the present invention, the antibody targeting TF of the second aspect of the present invention, or the multispecific antibody of the third aspect of the present invention;
(2) the recombinant protein of the fourth aspect of the present invention; and
(3) the CAR construct of the fifth aspect of the present invention.

In another preferred embodiment, the polynucleotide comprises RNA, DNA or cDNA.

In the eleventh aspect of the present invention, it provides a vector comprising the polynucleotide of the tenth aspect of the present invention.

In another preferred embodiment, the vector comprises: a bacterial plasmid, a phage, a yeast plasmid, a plant cell virus, a mammalian cell virus such as an adenovirus, retrovirus, or other vectors.

In the twelfth aspect of the present invention, it provides a genetically engineered host cell comprising the vector of the eleventh aspect of the present invention or having the polynucleotide of the tenth present aspect of the invention integrated into its genome.

In the thirteenth aspect of the present invention, it provides a method (including diagnostic or non-diagnostic method) for detection of TF in a sample *in vitro,* comprising the steps of:
(1) contacting the sample with the nanobody targeting TF of the first aspect of the present invention, the antibody targeting TF of the second aspect of the present invention, or the immunoconjugate of the seventh aspect of the present invention *in vitro;*
(2) detecting whether an antigen-antibody complex is formed, wherein the formation of the complex indicates the presence of TF in the sample.

In another preferred embodiment, the detection includes diagnostic or non-diagnostic detection.

In the fourteenth aspect of the present invention, it provides a detection plate comprising: a substrate (support plate) and a test strip, wherein the test strip contains the nanobody targeting TF of the first aspect of the present invention or the antibody targeting TF of the second aspect of the present invention or the immunoconjugate of the seventh aspect of the present invention.

In the fifteenth aspect of the present invention, it provides a kit comprising:
(1) a first container containing the nanobody targeting TF of the first aspect of the present invention or the antibody targeting TF of the second aspect of the present invention; and/or
(2) a second container containing a secondary antibody against the nanobody targeting TF of the first aspect of the present invention or the antibody targeting TF of the second aspect of the present invention;
alternatively, the kit contains the detection plate of the fourteenth aspect of the present invention.

In the sixteenth aspect of the present invention, it provides a method for preparing a recombinant polypeptide, comprising:
(a) culturing the host cell of the twelfth aspect of the present invention under a condition suitable for expression;
(b) isolating the recombinant polypeptide from the culture, wherein the recombinant polypeptide is the nanobody targeting TF of the first aspect of the present invention, the antibody targeting TF of the second aspect of the present invention, the multispecific antibody of the third aspect of the present invention, or the recombinant protein of the fourth aspect of the present invention.

In the seventeenth aspect of the present invention, it provides a method for treating a TF-related disease, comprising: administering the nanobody targeting TF of the first aspect of the present invention, the antibody targeting TF of the second aspect of the present invention, the multispecific antibody of the third aspect of the present invention, the recombinant protein of the fourth aspect of the present invention, the recombinant immune cell of the sixth aspect of the present invention, the immunoconjugate of the seventh aspect of the present invention, the pharmaceutical composition of the eighth aspect of the present invention, or a combination thereof to a subject in need thereof.

In another preferred embodiment, the method further comprises: administering other drugs or treatment methods to a subject in need thereof for combined treatment.

In another preferred embodiment, the other drugs or treatment methods include: anti-tumor immunotherapy drugs, tumor targeted drugs, tumor chemotherapy drugs, and tumor radiotherapy.

In another preferred embodiment, the anti-tumor immunotherapy drugs comprise PD-1 and PD-L1 monoclonal antibodies.

In the eighteenth aspect of the present invention, it provides a method for preparing a chimeric antibody, comprising the steps of:
cloning the nucleotide sequence of the alpaca-derived VHH sequence of the nanobody targeting TF of the first aspect of the present invention into an expression vector comprising the nucleotide sequence of the human antibody constant region, and expressing the human-alpaca chimeric antibody by transfecting animal cells.

In the nineteenth aspect of the present invention, it provides a method for preparing a humanized antibody, comprising the steps of:
the VHH chain of the nanobody targeting TF of the first aspect of the present invention is implanted into a nucleotide sequence template comprising the FR region of a human antibody, which is then cloned into an expression vector comprising a human antibody constant region, and the humanized antibody is expressed by transfecting animal cells.

In the twentieth aspect of the present invention, it provides a method for inhibiting tumor cell growth and migration, comprising the steps of administering the nanobody targeting TF of the first aspect of the present invention, the antibody targeting TF of the second aspect of the present invention, or the nanobody-drug conjugate of the present invention, or a CAR-T cell of the nanobody, or a combination thereof to a subject in need thereof.

In the twenty-first aspect of the present invention, it provides a method for inhibiting tumor growth in a model animal, comprising the steps of administering the nanobody targeting TF of the first aspect of the present invention, the antibody targeting TF of the second aspect of the present invention, the nanobody-drug conjugate of the present invention, or a CAR-T cell of the nanobody to a subject in need thereof.

In another preferred embodiment, the drug can be administered alone, or in combination with tumor immunotherapy, a tumor-targeted drug, a cytotoxic drug, or radiotherapy.

In the twenty-second aspect of the present invention, it provides a preparation method of the nanobody-drug conjugate of the seventh aspect of the present invention, comprising the steps of:
(1) reacting the antibody with a reducing agent in a buffer to obtain a reduced antibody;
(2) cross-linking (coupling) the reduced antibody obtained in step (1) with the linker-drug conjugate of formula DL in a mixture of buffer and organic solvent to obtain antibody-drug conjugates 1a, 1b, 1c or 1d with different DAR values.

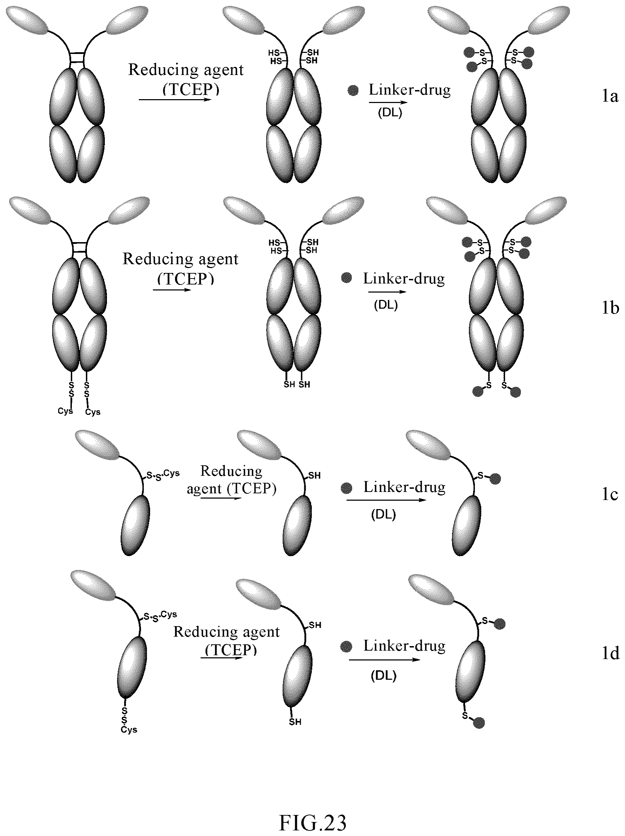

In another preferred embodiment, the cross-linking reaction of the preparation method is as shown in Figure 23.

In another preferred embodiment, the antibody in step (1) is reduced with a reducing agent, so that the interchain disulfide bonds of the antibody are reduced to generate thiol groups.

In another preferred embodiment, the reducing agent in step (1) is tris (2-carboxyethyl) phosphine hydrochloride (TCEP), beta-mercaptoethanol, beta-mercaptoethylamine hydrochloride, or dithiothreitol (DTT).

In another preferred embodiment, the buffer is selected from the group consisting of: potassium dihydrogen phosphate-sodium hydroxide (KH₂PO₄-NaOH)/sodium chloride (NaCl)/diethyltriaminepentaacetic acid (DTPA) buffer, disodium hydrogen phosphate-citric acid/sodium chloride (NaCl)/diethyltriaminepentaacetic acid (DTPA), boric acid-borax/sodium chloride (NaCl)/diethyltriaminepentaacetic acid (DTPA), histidine-sodium hydroxide/sodium chloride (NaCl)/diethyltriaminepentaacetic acid (DTPA), and PBS/diethyltriaminepentaacetic acid (DTPA).

In another preferred embodiment, in the step (2), the organic solvent in the reaction solution is no more than 15% by volume.

In another preferred embodiment, the organic solvent in step (2) is selected from the group consisting of: acetonitrile (ACN), dimethylformamide (DMF), dimethylacetamide (DMA), and dimethyl sulfoxide (DMSO).

Then the linker-drug (pre-dissolved in acetonitrile (ACN), dimethyl sulfoxide (DMSO), dimethylformamide (DMF) or diethylacetamide (DMA) at 10 mg/ml) is added. It should be ensured that the organic solvent in the reaction solution is no more than 15% by volume. The coupling reaction is processed under 0-37 °C and being stirred for 2-4 hours. If TCEP is used for reduction, it is also possible to directly add the substituted maleimide compound for coupling without removing the remaining TCEP.

The mixture of the coupling reaction is purified by filtration with sodium succinate/NaCl buffer or histidine-acetic acid/sucrose gel using a desalting column, and the peak samples are collected according to the UV280 ultraviolet absorption value. Or ultrafiltration is performed several times. Then, the product is filtered and sterilized, and stored at low temperature.

The drug-antibody conjugates obtained have a relatively uniform Drug-to-Antibody Ratio (DAR). For NDCs with certain differences in DAR, if a sample with better uniformity is required, further separation and purification can be performed by using methods including but not limited to: hydrophobic interaction chromatography (HIC), size exclusion chromatography (SEC), and ion exchange chromatography (IEC).

It should be understood that within the scope of the present invention, each technical feature of the present invention described above and in the following (such as examples) may be combined with each other to form a new or preferred technical solution, which is not listed here due to space limitations.

### DESCRIPTION OF THE DRAWINGS

Figure 1 shows the SDS-PAGE of human FC1 chimeric nanobodies after expression and purification, demonstrating the fusion proteins of the heavy chain variable region VHH of 4-A02, F1-A01, 4-C08, 4-E04, 4-C05, 5-C12, and 5-H3 fused with the FC1 fragment, respectively.
Figure 2 shows the binding affinity constant (KD) values of chimeric nanobodies 4-A02, F1-A01, 4-E04, 4-C05, 5-C12, and 5-H3 to human TF-ECD detected by surface plasmon resonance (SPR) analysis.
Figure 3 shows the binding affinity EC₅₀ detection results of 4-A02, F1-A01, 4-C08, 4-E04, 4-C05, 5-C12, and 5-H3 to TF on the surface of TF-high-expressing cell line MDA-MB-231.
Figure 4 shows the hydrophobic interaction chromatography (HIC) profile of the NDC of 4-A02-FC1 coupling to VC-MMAE conjugate.
Figure 5 shows the hydrophobic interaction chromatography (HIC) profile of the NDC of F1-A01-FC1 coupling to VC-MMAE conjugate.
Figure 6 shows the hydrophobic interaction chromatography (HIC) profile of the NDC of 4-C08-FC1 coupling to VC-MMAE conjugate.
Figure 7 shows the hydrophobic interaction chromatography (HIC) profile of the NDC of 4-C05-FC1 coupling to VC-MMAE conjugate.
Figure 8 shows the hydrophobic interaction chromatography (HIC) profile of the NDC of 5-C12-FC1 coupling to VC-MMAE conjugate.
Figure 9 shows the hydrophobic interaction chromatography (HIC) profile of the NDC of 4-E04-FC1 coupling to VC-MMAE conjugate.
Figure 10 shows the hydrophobic interaction chromatography (HIC) profile of the NDC of 4-A02-ABD coupling to VC-MMAE conjugate.
Figure 11 shows the hydrophobic interaction chromatography (HIC) profile of the NDC of 4-A02-FC1 coupling to VC-MMAE or GGFG-Dxd conjugate.
Figure 12 shows the detection results of the proliferation inhibitory activity of TF-NDC 4-A02-MMAE, F1-A01-MMAE, 4-E04-MMAE, 4-C05-MMAE, 5-C12-MMAE, 5-H3-MMAE, and the control HuSC1-MMAE on TF-high-expressing triple-negative breast cancer (TNBC) HCC1806 cells.
Figure 13 shows the detection results of the proliferation inhibitory activity of TF-NDC 4-A02-MMAE, F1-A01-MMAE, 4-E04-MMAE, 4-C05-MMAE, 5-C12-MMAE, 5-H3-MMAE, and the control HuSC1-MMAE on TF-high-expressing pancreatic ductal adenocarcinoma (PDAC) BxPC3 cells.
Figure 14 shows the detection results of the proliferation inhibitory activity of TF-NDC 4-A02-MMAE, F1-A01-MMAE, 4-E04-MMAE, 4-C05-MMAE, 5-C12-MMAE, 5-H3-MMAE, and the control HuSC1-MMAE on TF-high-expressing pancreatic ductal adenocarcinoma (PDAC) HPAF-II cells.
Figure 15 shows the detection results of the proliferation inhibitory activity of TF-NDC 4A02-FC1-MMAE, 4A02-FC1-DXd, and 4A02-ABD-MMAE against TF-high-expressing pancreatic ductal adenocarcinoma (PDAC) BxPC3 cells.
Figure 16 shows the detection results of the proliferation inhibitory activity of TF-NDC 4A02-FC1-MMAE, 4A02-FC1-DXd, and 4A02-ABD-MMAE on TF-high-expressing pancreatic ductal adenocarcinoma (PDAC) HPAF-II cells.
Figure 17 shows the detection results of the proliferation inhibitory activity of TF-NDC 4A02-FC1-MMAE, 4A02-FC1-DXd, and 4A02-ABD-MMAE on TF-abnormally expressed KRAS^{G12C}-mutated non-small cell lung cancer (NSCLC) HCC44 cells.
Figure 18 shows the detection results of the proliferation inhibitory activity of TF-NDC 4A02-FC1-MMAE, 4A02-FC1-DXd, and 4A02-ABD-MMAE on TF-abnormally expressed EGFR^{T790M}-mutated lung cancer NCI-H975 cells.
Figure 19 shows the detection results of the proliferation inhibitory activity of TF-NDC 4A02-FC1-MMAE, 4A02-FCWT-MMAE, 4A02-FC1-DXd, and 4A02-ABD-MMAE on TF-abnormally expressed EGFR-mutated lung cancer PC9 cells.
Figure 20 shows the SDS-PAGE of batch-produced proteins 4-A02-FC1, 4-A02-FCWT, 4-A02-FCLALAPG, and 4-A02-ABD after expression and purification, corresponding to SEQ NO.29, SEQ NO.30, SEQ NO.31, and SEQ NO.32.
Figure 21 shows the therapeutic effect of TF-NDC in nude mouse mammary pad triple negative breast cancer HCC1806 xenograft tumor growth model. Tumor bearing mice were grouped (n=8) on the 9th day and administered with hIgG-MMAE, 4-A02-FC1-MMAE, F1-A01-FC1-MMAE, 4-C08-FC1-MMAE, 4-C05-FC1-MMAE, 5-C12-FC1-MMAE, or 4-E04-FC1-MMAE via tail vein injection. The dosage was 3 mg/kg, with a single administration throughout the test.
Figure 22 shows the therapeutic effect of TF-NDC on KRAS^{G12C} HCC44 lung cancer orthotopic xenograft tumor growth model in nude mice. Tumor bearing mice were grouped (n=8) on the 13th day and administered with hIgG-MMAE or 4-A02-FC1-MMAE via tail vein injection. The dosage was 3 mg/kg, with a single administration throughout the test.
Figure 23 shows the cross-linking reaction equation of the preparation method of the nanobody-drug conjugate of the present invention, where antibody-drug conjugates with different DAR values are shown in 1a, 1b, 1c, or 1d, respectively.
Figure 24 shows the structural formulas of the fusion proteins of the present invention: (A) shows the structural formula of VHH-FC segment fusion protein (about 40kDa), (B) shows the structural formula of VHH-HLE fusion protein (about 30 kDa), and (C) shows the structural formula of VHH-ABD fusion protein (about 20 kDa).
Figure 25 shows the detection results of the proliferation inhibitory activity of 4A02-FC1-MMAE, 4A02-FCWT-MMAE, 4A02-FC1-DXd, and 4A02-ABCD-MMAE on TNBC cell line MDA-MB-231.
Figure 26 shows the detection results of the proliferation inhibitory activity of 4A02-FC1-MMAE, 4A02-FCWT-MMAE, 4A02-FC1-DXd, and 4A02-ABCD-MMAE on TNBC cell line HCC1806.
Figure 27 shows the detection results of the proliferation inhibitory activity of 4A02-FC1-MMAE, 4A02-FCWT-MMAE, 4A02-FC1-DXd, and 4A02-ABCD-MMAE on TNBC cell line HCC1954.
Figure 28 shows the detection results of the proliferation inhibitory activity of 4A02-FC1-MMAE, 4A02-FCWT-MMAE, 4A02-FC1-DXd, and 4A02-ABCD-MMAE on TNBC cell line HCC1937.
Figure 29 shows the detection results of the proliferation inhibitory activity of 4A02-FC1-MMAE, 4A02-FCWT-MMAE, 4A02-FC1-DXd, and 4A02-ABCD-MMAE on TNBC cell line MDA-MB-468.
Figure 30 shows the therapeutic effect of intravenous injection of 3mg/kg 4A02-FCWT-MMAE (once a week, twice in total) in the HCC1806 *in vivo* large tumor regression experiment.
Figure 31 shows the therapeutic effects of intravenous injection of 3mg/kg 4A02-FC1-MMAE, 4A02-FCWT-MMAE, 4A02-ABD-MMAE, and 10mg/kg 4A02-FC1-DXd (once a week, twice in total) in the HCC1954 *in vivo* model.
Figure 32 shows the therapeutic effect of TF-NDC on KRAS^{G12D} pancreatic cancer HPAF-II xenograft tumor growth model in nude mice. Figure 32A shows the therapeutic effect after a single intravenous injection of 3mg/kg 4A02-FC1-MMAE, 4A02-FCWT-MMAE, and 4A02-ABCD-MMAE. Figure 32B shows the therapeutic effect after a single intravenous injection of 1mg/kg 4A02-FC1-MMAE and 4A02-FCWT-MMAE.
Figure 33 shows the binding affinity constant (KD) values of humanized nanobodies 4A02-HM7, 4A02-HM8, 4A02-HM9 and the parental antibody 4A02 to human TF-ECD determined by surface plasmon resonance (SPR) analysis.
Figure 34 shows the binding affinity of humanized nanobodies 4A02-HM7, 4A02-HM8, 4A02-HM9 and the parental antibody 4A02 to TF on cell surface. Figure 34A shows the binding affinity to MDA-MB-231 cells detected by FACS. Figure 34B shows the binding affinity to HPAF-II cells detected by FACS.
Figure 35 shows the the affinity of humanized nanobodies 4A02-HM7, 4A02-HM8, 4A02-HM9 and the parental antibody 4A02 binding to TF-ECD detected by ELISA. Figure 35A shows the ELISA results of human TF-ECD. Figure 35B shows the ELISA results of cynomolgus monkey TF-ECD.

### DETAILED DESCRIPTION

Through extensive and intensive research, the inventors immunized alpaca and established libraries, screened phage display libraries and yeast display libraries in large quantities, and obtained multiple nanobodies such as 4-A02, F1-A01, 4-C08, 4-E04, 4-C05, 5-C12, and 5-H3. The antibodies can bind to human TF protein with high specificity, and the binding constant (KD) values detected by ForteBio surface plasmon resonance (SPR) ranges from 0.2nM to 7nM. The antibodies have excellent binding affinity for tumor cells with high TF expression, with EC₅₀ values ranging from 0.05 µg/mL to 0.75 µg/mL. In addition, the nanobody-drug conjugate designed based on TF nanobody (TF-NDC) can specifically kill tumor cells with moderate or high expression of TF, with IC₅₀ values ranging from 0.0001 µg/mL to 0.03 µg/mL. Single intravenous injection of 3mg/kg TF-NDC demonstrated excellent anti-tumor activity and potential to improve the immune microenvironment *in vivo.* The present invention was completed on this basis.

### Terms

As used herein, the terms "nanobody of the present invention", "anti-TF nanobody of the present invention", and "TF nanobody of the present invention" can be used interchangeably and refer to nanobodies that specifically recognize and bind to TF (including human TF). Particularly preferred are nanobodies with VHH chains having amino acid sequences as shown in SEQ ID NO.4, SEQ ID NO.8, SEQ ID NO.12, SEQ ID NO.16, SEQ ID NO.20, SEQ ID NO.24, and SEQ ID NO.28.

As used herein, the term "antibody" or "immunoglobulin" is a heterotetrameric glycoprotein of about 150,000 Da having the same structural characteristics, which is consisted of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by several constant regions. Each light chain has a variable region (VL) at one end and a constant region at the other end; the constant region of a light chain pairs with the first constant region of a heavy chain, and the variable region of a light chain pairs with the variable region of a heavy chain. Special amino acid residues form an interface between the variable regions of a light chain and a heavy chain.

As used herein, the terms "single domain antibody (VHH)" and "nanobody" have the same meaning and can be used interchangeably to refer to cloning the variable region of the heavy chain of the antibody, constructing a nanobody (VHH) composed of only one heavy chain variable region, which is the smallest antigen-binding fragment with complete function. Usually, the antibody with natural deletion of light chain and heavy chain constant region 1(CH1) is obtained first, and then the variable region of the antibody heavy chain is cloned to construct a nanobody (VHH) composed of only one heavy chain variable region.

As used herein, the term "variable" means that certain portion of the variable region in an antibody differ in sequence, which is responsible for the binding and specificity of various specific antibodies to their specific antigens. However, the variability is not distributed evenly throughout the variable regions of an antibody. It is concentrated in three segments called complementarity determining regions (CDRs) or hypervariable regions in the light and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partial β-sheet structure. The CDRs in each chain are closely linked together via the FR regions, and together with the CDRs of the other chain, form the antigen binding site of an antibody (see Kabat et al., NIH Publ. No. 91-3242, Vol. I, pp. 647-669 (1991)). The constant regions are not directly involved in the binding of an antibody to an antigen, however, they exhibit different effector functions, for example, involving in the antibody-dependent cytotoxicity of an antibody.

As known to those skilled in the art, immunoconjugates and fusion expression products include: conjugates formed by drugs, toxins, cytokines, radionuclides, enzymes, and other diagnostic or therapeutic molecules binding to the antibody or a fragment thereof of the present invention. The present invention also includes a cell surface marker or antigen that binds to the anti-TF protein antibody or a fragment thereof.

As used herein, the term "heavy chain variable region" can be used interchangeably with "V_{H}".

As used herein, the term "variable region" and "complementarity determining region (CDR)" can be used interchangeably.

In a preferred embodiment of the present invention, the heavy chain variable region of the antibody includes three complementary determining regions CDR1, CDR2, and CDR3.

In a preferred embodiment of the present invention, the heavy chain of the antibody includes the above-mentioned heavy chain variable region and heavy chain constant region.

In the present invention, the terms "antibody of the present invention", "protein of the present invention", or "polypeptide of the present invention" can be used interchangeably, and all refer to a polypeptide that specifically binds to TF protein, such as a protein or polypeptide having heavy chain variable regions. They may or may not contain a starting methionine.

The present invention further provides other proteins or fusion expression products having the antibody of the present invention. Specifically, the present invention includes any protein or protein conjugate and fusion expression product (i.e., immunoconjugate and fusion expression product) having a heavy chain containing a variable region, as long as the variable region is identical to or at least 90% homologous, preferably at least 95% homologous to the heavy chain variable region of the antibody of the present invention.

In general, the antigen-binding properties of an antibody can be described by three specific regions located in the variable region of the heavy chain, referred as variable regions (CDRs), and the segment is divided into four frame regions (FRs). The amino acid sequences of four FRs are relatively conservative and do not directly participate in binding reactions. These CDRs form a loop structure in which the β-sheets formed by the FRs in between are spatially close to each other, and the CDRs on the heavy chain and the CDRs on the corresponding light chain constitute the antigen-binding site of the antibody. The amino acid sequences of the same type of antibodies can be compared to determine which amino acids constitute the FR or CDR regions.

The variable regions of the heavy chains of the antibody of the present invention is particularly of interest because at least part of them is involved in binding to antigens. Thus, the present invention includes those molecules having an antibody heavy chain variable region with CDRs, provided that their CDRs are 90% or more (preferably 95% or more, and the most preferably 98% or more) identical to the CDRs identified herein.

### Anti-TF Nanobody

The present invention provides a variety of nanobodies targeting TF with high specificity and high affinity, which only comprise a heavy chain, and the heavy chain comprises heavy chain variable region (VH) amino acid sequences (listed in Table-1 and Table-2).

Preferably, the heavy chain variable region (VH) amino acid sequence has CDR1, CDR2, CDR3 of the following polypeptide sequences:
a1) CDR1 selected from:
   SEQ ID NO.1: ETISSTYI,
   SEQ ID NO.5: GRAFSAYA,
   SEQ ID NO.9: GFSLSSYD,
   SEQ ID NO.13: EMISSTYI,
   SEQ ID NO.17: GFTLDTYA,
   SEQ ID NO.21: GRTFSTDA, and
   SEQ ID NO.25: GFTLANYA;
a2) CDR2 selected from:
   SEQ ID NO.2: ISGDGVTH,
   SEQ ID NO.6: ISWSGGST,
   SEQ ID NO.10: IHSSGGYP,
   SEQ ID NO.14: ISGDGVTH,
   SEQ ID NO.18: ISSTDGST,
   SEQ ID NO.22: INWSGGST, and
   SEQ ID NO.26: ISRSDGDT;
a3) CDR3 selected from:
   SEQ ID NO.3: YAAGRWNH,
   SEQ ID NO.7: NADSLLSLLDGSRGGPGTDSGS,
   SEQ ID NO.11: NLPPSRRWYKDY,
   SEQ ID NO.15: NAAGRRNH,
   SEQ ID NO.19: AAGPGTDCPLRFDY,
   SEQ ID NO.23: VADSLLALLDGSRGGPGTDSDS, and
   SEQ ID NO.27: RATEWCGVQDPHGY.
a4) a sequence formed by adding, deleting, modifying and/or substituting at least one amino acid in any of the above amino acid sequences and having binding affinity to TF.

In another preferred embodiment, the sequence formed by adding, deleting, modifying and/or substituting at least one amino acid sequence is preferably an amino acid sequence with a homology of at least 80%, preferably at least 85%, more preferably at least 90%, and most preferably at least 95%.

Preferably, the antibody has the function of binding to or inhibiting the TF protein on cell surface and recombinant TF protein, and the antibody can be rapidly internalized into the lysosome by the cell.

The antibody of the present invention may be a double-chain or single-chain antibody, and may be selected from an animal-derived antibody, a chimeric antibody, a human-animal chimeric antibody, preferably a humanized antibody, and more preferably a fully humanized antibody.

The antibody derivative of the present invention may be a single-chain antibody and/or an antibody fragment, such as Fab, Fab', (Fab')2 or other antibody derivatives known in the art, as well as any one or more of IgA, IgD, IgE, IgG and IgM antibodies or other subtypes of antibodies.

Among them, the animal is preferably a mammal, such as mouse.

The antibody of the present invention may be a chimeric antibody, humanized antibody, CDR-grafted and/or modified antibody targeting human TF.

In a preferred embodiment of the present invention, any one or several sequences of the above-mentioned SEQ ID NO. 1~3, SEQ ID NO.5~7, SEQ ID NO.9~11, NO.13~15, NO.17~19, NO.21~23, and NO.25~27, or sequences formed by addition, deletion, modification and/or substitution of at least one amino acid with binding affinity to TF, are located in the CDR region of the variable region (VH).

In the above content of the present invention, the number of added, deleted, modified and/or substituted amino acids is preferably no more than 40%, more preferably no more than 35%, more preferably 1-33%, more preferably 5-30%, more preferably 10-25%, more preferably 15-20% of the total number of amino acids in the initial amino acid sequence.

In the above content of the present invention, more preferably, the number of the added, deleted, modified and/or substituted amino acids may be 1-7, more preferably 1-5, more preferably 1-3, more preferably 1-2.

In another preferred embodiment, the parental antibody is human-alpaca chimeric antibodies 4-A02, F1-A01, 4-C08, 4-E04, 4-C05, 5-C12, and 5-H3.

In another preferred embodiment, the amino acid sequence numbers of the heavy chain variable region (VH) of the chimeric antibodies are listed in Table 1.

In another preferred embodiment, the amino acid sequence numbers of the complementary determining region (CDR) of the antibody are listed in Table 2.

The antibody of the present invention may be used in combination, for the construction of a CAR construct, a recombinant immune cell comprising a CAR construct, an antibody-drug conjugate, etc., and may further be used for (a) preparing a detection reagent, a detection plate or kit; and/or (b) preparing a medicament for the prevention and/or treatment of a TF-related disease.

**Table-1: Representative meanings of each sequence in the sequence list of the present invention**

| Sequence No. | Sequence Name | Sequence No. | Sequence Name |
|---|---|---|---|
| SEQ ID NO.1 | 4-A02 CDR1 | SEQ ID NO.19 | 4-C05 CDR3 |
| SEQ ID NO.2 | 4-A02 CDR2 | SEQ ID NO.20 | 4-C05 VH |
| SEQ ID NO.3 | 4-A02 CDR3 | SEQ ID NO.21 | 5-C12 CDR1 |
| SEQ ID NO.4 | 4-A02 VH | SEQ ID NO.22 | 5-C12 CDR2 |
| SEQ ID NO.5 | F1-A01 CDR1 | SEQ ID NO.23 | 5-C12 CDR3 |
| SEQ ID NO.6 | F1-A01 CDR2 | SEQ ID NO.24 | 5-C12 VH |
| SEQ ID NO.7 | F1-A01 CDR3 | SEQ ID NO.25 | 5-H3-FC1 |
| SEQ ID NO.8 | F1-A01 VH | SEQ ID NO.26 | 5-H3-FC2 |
| SEQ ID NO.9 | 4-C08 CDR1 | SEQ ID NO.27 | 5-H3-FC3 |
| SEQ ID NO.10 | 4-C08 CDR2 | SEQ ID NO.28 | 5-H3 VH |
| SEQ ID NO.11 | 4-C08 CDR3 | SEQ ID NO.29 | 4-A02-FC1 |
| SEQ ID NO.12 | 4-C08 VH | SEQ ID NO.30 | 4-A02-FCWT |
| SEQ ID NO.13 | 4-E04 CDR1 | SEQ ID NO.31 | 4-A02-FCLALAPG |
| SEQ ID NO.14 | 4-E04 CDR2 | SEQ ID NO.32 | 4-A02-ABD |
| SEQ ID NO.15 | 4-E04 CDR3 | SEQ ID NO.33 | 4A02-HM7-FCWT |
| SEQ ID NO.16 | 4-E04 VH | SEQ ID NO.34 | 4A02-HM8-FCWT |
| SEQ ID NO.17 | 4-C05 CDR1 | SEQ ID NO.35 | 4A02-HM9-FCWT |
| SEQ ID NO.18 | 4-C05 CDR2 | | |

**Table-2: CDR amino acid seauences of nanobodies of the resent invention**

| TF Nanobody | CDR1 | CDR2 | CDR3 |
|---|---|---|---|
| 4-A02 | ETISSTYI | ISGDGVTH | YAAGRWNH |
| F1-A01 | GRAFSAYA | ISWSGGST | NADSLLSLLDGSRGGPGTDSGS |
| 4-C08 | GFSLSSYD | IHSSGGYP | NLPPSRRWYKDY |
| 4-E04 | EMISSTYI | ISGDGVTH | NAAGRRNH |
| 4-C05 | GFTLDTYA | ISSTDGST | AAGPGTDCPLRFDY |
| 5-C12 | GRTFSTDA | INWSGGST | VADSLLALLDGSRGGPGTDSDS |
| 5-H3 | GFTLANYA | ISRSDGDT | RATEWCGVQDPHGY |

### Recombinant protein (or fusion protein)

In the present invention, it further comprises a recombinant protein (or fusion protein) comprising the TF nanobody of the present invention. A preferred fusion protein is a multispecific antibody, which further comprises a second antigen binding region targeting a target selected from the group consisting of: EGFR, TGFβ, BCMA, B7H6, GUCY2C, DLL3, CD38, CD123, CD19, CD20, CD22, B7-H3, GPC3, HER2, PMSA, CD28, 4-1BB, OX40, CD40, CD27, CD3, CTLA4, PD1, PDL1, CD73, BCMA, GLP-1, Trop2, TIGIT, LAG-3, FGL1, TLR7, and a combination thereof.

Preferably, the multispecific antibody comprises one or more second antigen binding regions, or further comprises a third antigen binding region.

Furthermore, the recombinant protein (or fusion protein) of the present invention, in addition to the CD73 nanobody of the present invention, further comprises an optional tag sequence that assists in expression and/or purification (e.g., 6His tag, GGGS sequence, FLAG tag); or comprises an optional polypeptide molecule or fragment with therapeutic function; or an optional protein functional domain that assists in physicochemical or pharmaceutical properties (e.g., molecules that may prolong the *in vivo* half-life of a nanobody, such as HLE, ABD).

The present invention includes not only intact antibodies but also fragments of immunologically active antibody or fusion proteins formed from antibodies with other sequences. Therefore, the present invention also includes fragments, derivatives and analogs of the antibodies.

As used herein, the terms "fragment", "derivative", and "analog" refer to a polypeptide that substantially retains the same biological function or activity of a antibody of the invention. Polypeptide fragments, derivatives or analogs of the invention may be (i) polypeptides having one or more conservative or non-conservative amino acid residues (preferably conservative amino acid residues) substituted, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence or sequence or protein sequence used to purify the polypeptide, or a fusion protein formed with a 6His tag). According to the teachings herein, these fragments, derivatives and analogs are within the scope of well-known to those skilled in the art.

The antibody of the present invention refers to a polypeptide having TF protein binding activity and comprising the CDR regions as described above. The term also comprises variant forms of polypeptides having the same function as the antibody of the present invention and comprising the CDR regions as described above. These variants include (but are not limited to): deletion, insertion and/or substitution of one or more (usually 1-50, preferably 1-30, more preferably 1-20, most preferably 1-10) amino acids, and addition of one or more (usually within 20, preferably within 10, more preferably within 5) amino acids at the C-terminal and/or N-terminal. For example, in the art, substitutions with amino acids of similar properties generally do not alter the function of the protein. For another example, addition of one or more amino acids to the C-terminal and/or N-terminal usually does not alter the function of the protein. The term also includes active fragments and active derivatives of the antibody of the present invention.

The variant forms of the polypeptide include homologous sequences, conservative variants, alleles, natural mutants, induced mutants, proteins encoded by DNA capable of hybridizing with the coding DNA of the antibody of the present invention under high or low tightness conditions, and polypeptides or proteins obtained by using anti-serum against the antibody of the present invention.

The present invention also provides other polypeptides, such as a fusion protein containing a nanobody or a fragment thereof. In addition to the almost full-length polypeptide, the present invention also includes a fragment of the nanobody of the present invention. Typically, the fragment has at least about 50 contiguous amino acids, preferably at least about 50 contiguous amino acids, more preferably at least about 80 contiguous amino acids, and most preferably at least about 100 contiguous amino acids of the antibody of the present invention.

In the present invention, "conservative variant of the antibody of the present invention" refers to a polypeptide formed by substituting at most 10, preferably at most 8, more preferably at most 5, and most preferably at most 3 amino acids with amino acids of similar properties as compared with the amino acid sequence of the antibody of the present invention. These conservative variant polypeptides are best produced by amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the above antibody or fragment thereof or fusion protein thereof. The polynucleotide of the present invention may be in the form of DNA or RNA. DNA form includes cDNA, genomic DNA, or synthetic DNA. DNA may be single-stranded or double-stranded. DNA may be a coding strand or a non-coding strand.

The polynucleotide encoding the mature polypeptide of the present invention includes: the coding sequence that encodes only the mature polypeptide; the coding sequence of the mature polypeptide and various additional coding sequences; the coding sequence of the mature polypeptide (and optional additional coding sequence) and the non-coding sequence.

The term "polynucleotide encoding a polypeptide" may be a polynucleotide that includes sequence encoding the polypeptide, or a polynucleotide that also includes additional coding and/or non-coding sequences.

The present invention also relates to a polynucleotide that hybridizes to the sequence as described above and has at least 50%, preferably at least 70%, and more preferably at least 80% identity between the two sequences. In particular, the present invention relates to a polynucleotide that is hybridizable to the polynucleotide of the present invention under strict conditions. In the present invention, "strict condition" refers: (1) hybridization and elution at lower ionic strength and higher temperature, such as 0.2×SSC, 0.1% SDS, 60°C; or (2) hybridiztion with denaturing agent, such as 50%(v/v) formamide, 0.1% calf serum/0.1% Ficoll, 42°C, etc.; or (3) hybridization occurs only when the identity between the two sequences is at least 90% or more, more preferably 95% or more. Furthermore, the polypeptide encoded by the hybridizable polynucleotide has the same biological function and activity as the mature polypeptide.

The full-length nucleotide sequence or fragments of the antibody of the present invention may generally be obtained by PCR amplification, recombination or artificial synthesis methods. A feasible method is to synthesize the relevant sequence by artificial synthesis, especially when the fragment length is short. Generally, fragments with a long sequence may be obtained by first synthesizing multiple small fragments followed by ligation. In addition, the coding sequence of the heavy chain and the expression tag (such as 6His) may be fused together to form a fusion protein.

### Preparation of Antibody

The sequence of the DNA molecule for the antibody or a fragment thereof of the present invention may be obtained by conventional techniques, for example, methods such as PCR amplification or genomic library screening. In addition, the sequences encoding light chain and heavy chain may be fused together, to form a single-chain antibody.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods.

In addition, a relevant sequence may be synthesized artificially, especially when the fragment is short in length. Usually, several small fragments are synthesized first, and then are linked together to obtain a fragment with a long sequence.

At present, it is possible to obtain a DNA sequence encoding the antibody of the present invention (or fragments thereof, or derivatives thereof) completely by chemical synthesis. The DNA sequence can then be introduced into a variety of existing DNA molecules (or, for example, vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors may be used to transform suitable host cells to enable them to express protein.

The host cell may be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody of the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained may be identified by conventional means. For example, the binding specificity of a monoclonal antibody may be determined by immunoprecipitation or an in vitro binding assay (such as radioimmunoassay (RIA) or enzyme-linked immunosorbent assay (ELISA)). The binding affinity of a monoclonal antibody may be determined by, for example, the Scatchard analysis (Munson et al., Anal. Biochem. 107: 220 (1980)).

The antibody according to the present invention may be expressed in a cell or on the cell membrane, or be secreted extracellularly. If necessary, the recombinant protein may be separated and purified by various separation methods according to its physical, chemical, and other properties. These methods are well known to those skilled in the art. The examples of these methods comprise, but are not limited to, conventional renaturation treatment, treatment by protein precipitant (such as salt precipitation), centrifugation, cell lysis by osmosis, ultrasonic treatment, super centrifugation, molecular sieve chromatography (gel chromatography), adsorption chromatography, ion exchange chromatography, high performance liquid chromatography (HPLC), and any other liquid chromatography, and a combination thereof.

### Nanobody-drug conjugate (NDC)

The present invention also provides an immunoconjugate (ADC) based on the antibody of the present invention, preferably a nanobody-drug conjugate (NDC).

Typically, the antibody-drug conjugate comprises the antibody and an effector molecule, wherein the antibody is conjugated to the effector molecule, and chemical conjugation is preferred. Wherein, the effector molecule is preferably a therapeutically active drug. In addition, the effector molecule may be one or more of a toxic protein, a chemotherapeutic drug, a small-molecule drug or a radionuclide.

The antibody according to the present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that links an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly (ethylene glycol)) and therapeutic agents. An antibody may be conjugated to a functional agent to form a conjugate of the antibody-functional agent. A functional agent (e.g., a drug, a detection reagent, a stabilizer) is conjugated (covalently linked) to an antibody. A functional agent may be linked to an antibody either directly or indirectly via a linker.

Nanobodies may be conjugated to drugs to form antibody-drug conjugates (NDCs). Typically, a NDC comprises a linker between a drug and an antibody. The linker may be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that may be degraded by protease (e.g., lysosomal protease or endosomal protease) in a cell, or sugar linkers, for example, glucuronide-containing linkers that may be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g., linkers that are hydrolyzed when the pH is less than 5.5, such as hydrazone linkers) and linkers that are degraded under a reducing condition (e.g., disulfide-bond linkers). A non-degradable linker typically releases a drug under a condition that the antibody is hydrolyzed by a protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g., iodo-, bromo- or chloro-substituted) amide; a halogenated (e.g., iodo-, bromo- or chloro-substituted) ester; a halogenated (e.g., iodo-, bromo- or chloro-substituted) methyl ketone, a benzyl halide (e.g., iodide, bromide or chloride); vinyl sulfone, pyridyl disulfide; a mercury derivative such as 3,6-di-(mercurymethyl)dioxane, wherein the counter ion is acetate ion, chloride or nitrate ion; and polymethylene dimethyl sulfide thiosulfonate. The linker may include, for example, a maleimide linked to an antibody via thiosuccimide.

Drug may be any cytotoxic drug which inhibits cell growth or immunosuppression. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, immune agonist, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, duocarmycins, etoposides, maytansines and maytansinoids (e.g., DM1 and DM4), taxanes, benzodiazepines or benzodiazepine containing drugs (e.g., pyrrolo[1,4]benzodiazepines (PBDs), indolinobenzodiazepines and oxazolidinobenzodiazepines), and vinca alkaloids.

In the present invention, a drug-linker may be used to form a NDC in a simple step. In other embodiments, a bifunctional linker compound may be used to form a NDC in a two-step or multi-step process. For example, a cysteine residue is reacted with the reactive moiety of a linker in a first step, and then the functional group on the linker is reacted with a drug in the subsequent step, thereby forming a NDC.

In general, the functional group on a linker is selected so that it can specifically react with the suitable reactive group on a drug moiety. As a non-limiting example, an azide-based moiety may be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in "Bioconjugation Technology" (2nd Edition (Elsevier)), are well known to those skilled in the art. Those skilled in the art could understand that when a complementary pair of reactive functional groups is selected for a selective reaction between a drug moiety and a linker, each member of the complementary pair may be used for the linker, and may also be used for the drug.

Preferably, the coupling moiety of the nanobody drug conjugate (NDC) in the present invention that is coupled to the nanobody moiety comprises a protein degradation agent or an oligonucleotide drug.

Preferably, the oligonucleotide drug is a nucleic acid drug targeting small molecules, including antisense nucleic acid (ASO), small interfering RNA (siRNA), microRNA (miRNA), small activating RNA (saRNA), messenger RNA (mRNA), aptamer, ribozyme, antibody nucleic acid conjugate drug (ARC), etc.

As used herein, the protein degradation agent is a degradation agent for tumor associated proteins selected from the group consisting of:
(1) kinases such as RIPK2, BCR-ABL, EGFR, HER2, c-Met, TBK1, CDK2/4/6/9, ALK, Akt, CK2, ERK1/2, FLT3, PI3K, BTK, Fak, etc;
(2) BET proteins, such as BRD2/4/6/9;
(3) nuclear receptors such as AR, ER, etc.;
(4) other proteins, such as MetAp-2, Bcl-xL, Sirt2, HDAC6, Pirin, SMAD3, ARNT, PCAF/GCN5, Tau, FRS2, RAS (KRAS, HRAS, and NRAS), etc.

Preferably, the protein degrader comprises PROTAC (Protein Degradation Targeting Chimera), and more preferably, the PROTAC targets EGFR, KRAS (e.g., KRAS-G12C, KRAS-G12D).

The present invention further provides a method for preparing an ADC, which may further comprise: under a condition sufficient to form an antibody-drug conjugate (NDC), binding an antibody to a drug-linker compound.

In certain embodiments, the method according to the present invention comprises: under a condition sufficient to form an antibody-linker conjugate, binding an antibody to a bifunctional linker compound. In these embodiments, the method according to the present invention further comprises: under a condition sufficient to covalently link the drug moiety to the antibody via a linker, binding the antibody-linker conjugate to the drug moiety.

In some embodiments, a nanobody-drug conjugate (NDC) has a formula as follows: 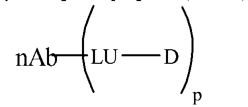 wherein:
nAb is a nanobody,
LU is a linker/connector;
D is a drug;
and the subscript p is a value selected from1 to 8.

### Drug

As used herein, the term "drug" refers to any compound possessing a desired biological activity and a reactive functional group available for preparing the conjugate of the present invention. The desired biological activity includes activity useful in the diagnosis, cure, mitigation, treatment, or prevention of a disease in human or other animals. Thus, so long as it has the needed reactive functional group, the compound involved by the term "drug" include drugs identified in the official national pharmacopeia as well as e.g., official Homeopathic Pharmacopeia of the United States, or official National Formulary, or any supplements thereof. Exemplary drugs are set forth in the Physician's Desk Reference (PDR) and in the Orange Book maintained by the U.S. Food and Drug Administration (FDA). It should be understood that, as new drugs are continually discovered and developed, these drugs shall also be incorporated into the "drug" of the drug conjugates of the present invention.

The drugs useful to constitute the NDC of the present invention include, but are not limited to, cytotoxic agents (such as small molecule cytotoxic drugs).

The term "cytotoxic agents" refer to substances that inhibit or block cell expression activity, cell function and/or result in cell destruction. The term includes radioisotopes, chemotherapeutics, and toxins, such as small-molecular toxins or enzymatically active toxins (including fragments and/or variants thereof) derived from bacteria, fungi, plants or animals. Examples of cytotoxic agents include, but are not limited to: auristatins (for example, auristatin E, auristatin F, MMAE and MMAF), chlortetracycline, maytansinoid, ricin, ricin A-chain, cobustatin, dokamicin, dorastatin, adriamycin, daunorubicin, paclitaxel, cisplatin, cc1065, ethidium bromide, mitomycin, etoposide, tenoposide, vincristine, vinblastine, colchicine, dihydroxyanthracnose diketone, actinomycin, diphtheria toxin, Pseudomonas exotoxin (PE) A, PE40, abrin, abrin A chain, modeccin A chain, α-Sarcina, gelonin, mitogellin, retstrictocin, phenomycin, enomycin, curicin, crocotin, calicheamicins, Sapaonaria officinalis inhibitor, as well as glucocorticoid and other chemotherapy agents, as well as radioisotopes such as At211, I131, I125, Y90, Re186, Re188, Sm153, Bi212 or Bi213, P32 and Lu (including Lu177). Antibodies can also be conjugated to anticancer prodrug activating enzymes that can convert a prodrug into the active form.

One preferred drug useful in the present invention is maytansine or maytansinoids. Maytansine inhibits cell proliferation by inhibiting the formation of microtubules from tubulins. Maytansinoids are derivatives of maytansine. Both maytansine and maytansinoids are highly cytotoxic, but they are greatly limited for clinical use in cancer therapy due to poor selectivity for tumors. However, the high cytotoxicity enables them to be attractive drug moieties in antibody-drug conjugates. The structure shown below is deacetyl-maytansine.

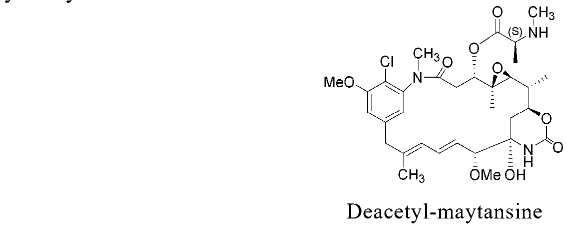

Another preferred drug useful in the present invention is auristatins. Auristatins are synthetic analogues of dolastatin 10, which is a polypeptide isolated from marine mollusk Aplysia having biological activity. Dolastatin 10 inhibits tubulin polymerization by binding to tubulin at the same domain as anticancer drug vincristine. Dolastitin 10, auristatin PE, and auristatin E are all linear peptides having four amino acids, three of which are unique to the dolastatin class compounds, and a C-terminal amide group. Two representative auristatins, monomethyl auristatin E (MMAE) and monomethyl auristatin F (MMAF), are preferred drug moiety candidates for ADC.

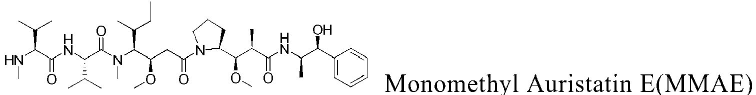

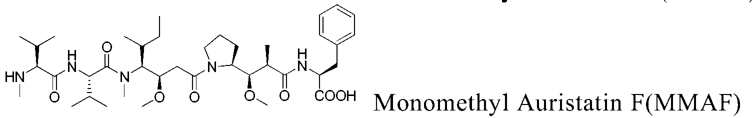

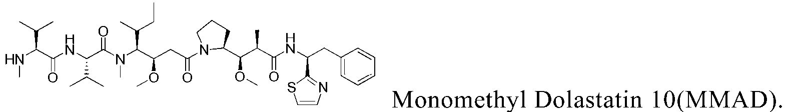

Another preferred drug of the present invention is microtubuleysin (tubulysin). Microtubuleysin was first isolated by the research team from slime cell cultures and is a highly effective cell growth inhibitor that works by inhibiting microtubule protein polymerization and inducing cell apoptosis. Microtubuleysin D in microtubuleysins is the most effective, with activity 10 to 100 times higher than most other microtubule protein regulators, including erythromycin, vinblastine, and paclitaxel. Paclitaxel and vinblastine are currently used for the treatment of various cancers, while epomycin derivatives are undergoing activity evaluation in clinical trials. The synthetic derivatives of microtubuleysin D will provide necessary information related to inhibition and critical binding interactions, and can exhibit superior properties as anticancer agents, either as isolated entities or as chemical warheads on targeted antibodies or ligands. Microtubuleysin D is a complex tetrapeptide that may be divided into four regions: Mep (D-N-methylpiperidinecarboxylic acid), Ile (isoleucine), Tuv (tubulvaline), and Tup (tubulphenylalanine), as shown in the following formula:

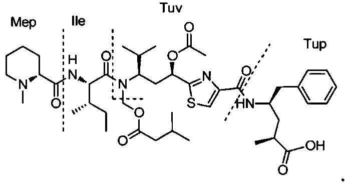

Another preferred drug of the present invention is a cryptophycin derivative derived from microorganisms that can inhibit microtubule polymerization. Cryptophycin is a novel anti-tumor active substance isolated from blue-green algae cultures that can inhibit microtubule formation and has activity against various tumors. Cryptophycin is a lipophilic compound containing 2 peptide bonds and 2 ester bonds, with 5 optically active centers and 1 epoxy group. The dipeptide diester bonds are all in a macrocyclic structure. The structures of Cryptophycin derivatives CP1 and CP2 are shown in the following formulas:

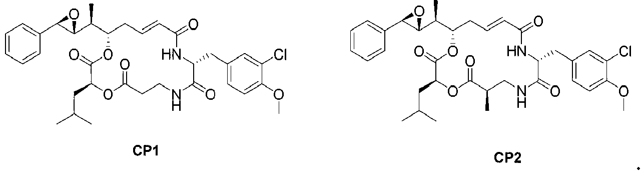

Another preferred drug of the present invention is the novel microtubule inhibitor Taltobulin (HTI-286, SPA-110). Taltobulin inhibits the polymerization of purified microtubules, interferes with intracellular microtubule organization, induces mitotic blockade, and induces cell apoptosis. Taltobulin is a potent inhibitor of cell proliferation, with an average IC50 of 2.5 nM against 18 human tumor cell lines. Compared with the currently used microtubule inhibitors, Taltobulin is not a suitable substrate for p-glycoprotein, and its structure is shown in the following figure.

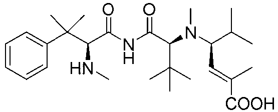

In one aspect, the drug is a derivative of camptothecin, SN-38. SN-38 is a bioactive metabolite of irinotecan hydrochloride (CPT-11) and a class of topoisomerase inhibitors. SN-38 causes the strongest inhibition of DNA topoisomerase I, inhibits DNA synthesis in dose- and time-dependent manners, and causes frequent DNA single strand breaks. The structure of SN-38 is shown in the following figure.

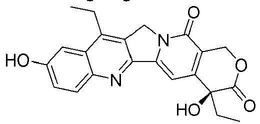

In one aspect, the drug is Exatecan, a derivative of camptothecin. It is a synthetic analogue of the topoisomerase I inhibitor camptothecin, with stronger activity than SN-38. It can cause the strongest inhibition of DNA topoisomerase I, inhibits DNA synthesis in dose- and time-dependent manners, and causes frequent DNA single strand breaks. The structure of Exatecan is shown in the following formula.

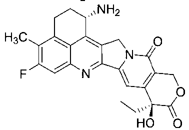

Another preferred drug of the present invention is alpha-Amanitin, which has the structure shown in the following figure. Alpha-Amanitin is a fungal toxin derived from Amanita phalloides, which is a poisonous mushroom. It is a bicyclic octapeptide and can inhibit the transcription of eukaryotic RNA polymerase II and RNA polymerase III.

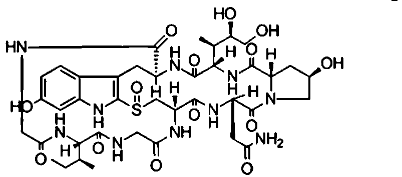

Another preferred drug of the present invention is a benzodiazepine antibiotic (duocarmycins, CC-1065, etc.) and other cyclopropyrrolidind-4-one (CPI) derivatives. This type of compound is an effective DNA small groove binding alkylating reagent. Cyclopropabenzindol-4-one (CBI) analogues have more stable chemical structures, higher biological activity, and are easier to be synthesized compared to their parent compounds containing natural CPI alkylated subunits. A representative CBI derivative is the phenolic hydroxyl protected derivative CBI, which has weakened prodrug toxicity and enhanced water solubility (wherein the structural formula of CBI-seco class is shown in the following figure):

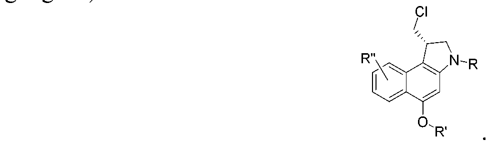

Another preferred drug of the present invention is pyrrolo[2,1-c][1,4]benzodi-azepine (PBDs) or PBD dimers. PBD is a natural product produced by *Streptomyces,* characterized by the ability to form non twisted covalent adducts in DNA grooves, specifically at the purine-guanine-purine sequence. The application of PBD as a partial small molecule strategy to target and lock DNA sequences and as a novel anti-cancer and antibacterial drug has attracted increasing interest. The application of a flexible carbon chain to connect the C8/C8' hydroxyl groups of two PBD units results in a dimer with enhanced biological activity. PBD dimers are believed to generate sequence selective DNA damage, such as reverse 5'-Pu-GATC-Py-3' interchain cross-linking, leading to their biological activity. These compounds have been proven to be highly effective cytotoxic drugs and can serve as alternative drugs for antibody drug conjugates.

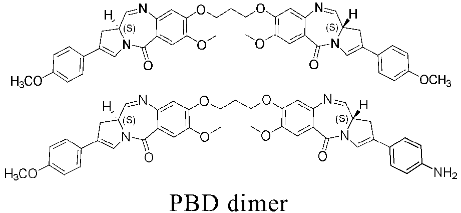

Another preferred drug of the present invention is a derivative of PNU-159682. PNU-159682 is the main active metabolite of Nemorubicin in human liver microsomes. Compared with MMDX and doxorubicin, PNU-159682 exhibits a 3000 folds increase in activity.

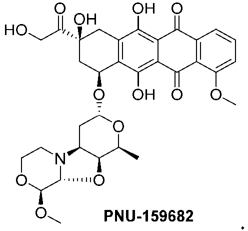

On the other hand, drugs are not limited to the categories mentioned above, but also include all drugs that may be used for antibody drug conjugates. And especially those that can coordinate through amide bonds with the linker, such as cell toxins that coordinate through basic amino groups (primary or secondary amines), such as the structure of cell toxins D1-D12 shown above.

### Linker

According to the mechanism of drug release within cells, "linker" or "linker of antibody drug conjugate" may be divided into two categories: unbreakable linker and breakable linker.

For antibody drug conjugates containing unbreakable linkers, the drug release mechanism is as follows: after the conjugate binds to the antigen and is internalized by the cell, the antibody is enzymatically hydrolyzed in the lysosome, releasing an active molecule composed of small molecule drugs, linkers, and antibody amino acid residues. The resulting changes in the molecular structure of the drug do not weaken its cytotoxicity, but due to the charged nature of the active molecule (amino acid residues), it cannot penetrate into neighboring cells. Therefore, such active drugs cannot kill adjacent tumor cells that do not express the targeted antigen (antigen negative cells) (bystander effect).

The breakable linker, as the name suggests, can break and release active drugs (small molecule drugs themselves) within the target cell. Breakable linkers may be divided into two main categories: chemically unstable linkers and enzymatically unstable linkers. Chemical unstable linkers can selectively break due to differences in plasma and cytoplasmic properties. Such properties include pH value, glutathione concentration, etc. The pH sensitive linkers are commonly referred to as acid cleaved linkers. Such linkers are relatively stable in the neutral environment of blood (pH 7.3-7.5), but will be hydrolyzed in weakly acidic endosomes (pH 5.0-6.5) and lysosomes (pH 4.5-5.0). The first generation of antibody drug conjugates mostly used such linkers, such as hydrazones, carbonates, acetals, and ketones. Due to the limited plasma stability of acid cleaved linkers, antibody drug conjugates based on such linkers typically have a short half-life (2-3 days). This relatively short half-life to some extent limits the application of pH sensitive linkers in the new generation of antibody drug conjugates.

For glutathione sensitive linker, it is also known as disulfide bond linker. Drug release is based on the difference between the high concentration of intracellular glutathione (in the millimolar range) and the relatively low concentration of glutathione in the blood (in the micromolar range). This is especially true for tumor cells, whose low oxygen content leads to increased activity of reductases, resulting in higher concentrations of glutathione. Disulfide bond has thermodynamic stability and therefore exhibits good stability in plasma.

Enzyme unstable linkers, such as peptide linkers, can better control drug release. Peptide linkers may be effectively cleaved by proteases within the lysosome, such as cathepsin B or plasmin (which have increased levels in some tumor tissues). This peptide linkage is considered to be very stable in plasma circulation, as inappropriate pH values outside the cell and serum protease inhibitors often render proteases inactive. Due to its high plasma stability and good intracellular cleavage selectivity and effectiveness, enzyme unstable linkers are widely used as breakable linkers for antibody drug conjugates. Typical enzyme unstable linkers include Val-Cit (VC), Val-Ala (VA), Gly-Gly-Phe-Gly (GGFG), etc.

The self-releasing linker is generally embedded between the breakable linker and the active drug, or is itself a part of the breakable linker. The action mechanism of the self-releasing linker is that when the breakable linker is broken under appropriate conditions, the self-releasing linker can spontaneously undergo structural rearrangement, thereby releasing the active drug connected to it. Common self-releasing linkers include para-aminobenzyl alcohol (PAB) and β-glucuronide, etc.

### Application

The present invention also provides a use of the antibodies of the present invention, such as for preparing diagnostic preparation, or for preparing medicine for preventing and/or treating TF-related diseases. The TF-related diseases include tumor occurrence, growth and/or metastasis, tumor resistance related diseases, inflammation, metabolism-related diseases, etc.

Use of the antibodies, NDCs or CAR-T cells of the present invention includes (but is not limited to):
(i) diagnosis, prevention and/or treatment of tumorigenesis, tumor growth and/or metastasis, particularly, for a tumor with a TF high expression; wherein the tumor includes (but is not limited to): breast cancer (such as triple negative breast cancer), lung cancer (such as non-small cell lung cancer), pancreatic cancer, malignant glioma, gastric cancer, liver cancer, esophageal cancer, kidney cancer, colorectal cancer, bladder cancer, prostate cancer, endometrial cancer, ovarian cancer, cervical cancer, leukemia, bone marrow cancer, angiosarcoma, etc.; preferably triple negative breast cancer, non-small cell lung cancer, pancreatic cancer, malignant glioma; and more preferably triple negative breast cancer, non-small cell lung cancer, pancreatic cancer, etc.;
(ii) diagnosis, prevention and/or treatment of an autoimmune disease; wherein the autoimmune disease includes (but is not limited to): systemic lupus erythematosus, rheumatoid arthritis, ulcerative colitis, type I diabetes, psoriasis, multiple sclerosis;
(iii) diagnosis, prevention and/or treatment of inflammation; wherein the inflammation includes (but is not limited to): rheumatic arthritis, osteoarthritis, ankylosing spondylitis, gout, Lytle syndrome, psoriasis arthritis, infectious arthritis, tuberculous arthritis, viral arthritis, fungal arthritis, glomerular nephritis, systemic lupus erythematosus, Crohn's disease, ulcerative colitis, acute lung injury, chronic obstructive pulmonary disease, and idiopathic pulmonary fibrosis;
(iv) diagnosis, prevention and/or treatment of a metabolism-related disease, wherein the metabolism-related disease includes (but is not limited to): diabetes, diet-induced obesity, adipose inflammation.

### Pharmaceutical composition

The present invention further provides a composition. In the preferred examples, the composition is a pharmaceutical composition comprising the above antibody, or an active fragment, a fusion protein or a NDC thereof, or a corresponding CAR-T cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, although the pH value may be varied depending on the nature of the substances to be formulated and the condition to be treated. The formulated pharmaceutical composition may be administered by conventional routes, including (but not limited to): intratumoral, intraperitoneal, intravenous, or topical administration.

The antibody of the present invention can also be used for cell therapy by expressing the nucleotide sequence in a cell, for example, the antibody is used for chimeric antigen receptor T cell immunotherapy (CAR-T) and the like.

The pharmaceutical composition of the present invention can be directly used for binding to TF protein molecule, and thus can be used for preventing and treating diseases such as tumors. In addition, other therapeutic agents can also be used simultaneously.

The pharmaceutical composition according to the present invention comprises a safe and effective amount (e.g., 0.001-99 wt%, preferably 0.01-90 wt%, preferably 0.1-80 wt%) of the monoclonal antibody according to the present invention (or a conjugate thereof) and a pharmaceutically acceptable carrier or excipient. Such carriers include, but are not limited to, saline, buffer solution, glucose, water, glycerin, ethanol or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 1 µg/kg body weight to about 5 mg/kg body weight per day. Further, the polypeptide of the present invention can also be used in combination with the other therapeutic agents.

When a pharmaceutical composition is used, a safe and effective amount of the immunoconjugate is administered to a mammal, wherein the safe and effective amount is usually at least about 10 µg/kg body weight, and in most cases does not exceed about 50 mg/kg body weight, preferably the dose is about 10 µg/kg body weight to about 20 mg/kg body weight. Of course, the particular dose should also depend on various factors, such as the route of administration, patient healthy status, which are well within the skills of an experienced physician.

As for NDC, the nanobody-drug conjugate provided by the present invention can target a specific cell population and bind to a specific protein (antigen) on cell surface, thereby releasing the drug into the cell in an active form through endocytosis or drug infiltration of the conjugate. Thus, the nanobody-drug conjugate of the invention can be used to treat diseases of interest, and the antibody-drug conjugate mentioned above can be administered to a subject (e.g., a human) by a suitable route in a therapeutically effective amount. A subject in need of treatment can be a patient at risk of having or suspected of having a condition associated with the activity or amount of expression of a particular antigen. Such patients can be identified by routine physical examination.

When the nanobody-drug conjugate as described herein is used as the therapeutic agent, it can be delivered by methods conventional in the art. For example, it can be introduced into cells using liposomes, hydrogels, cyclodextrins, biodegradable nanocapsules, or bioadhesive microspheres. Alternatively, nucleic acids or vectors can be delivered *in situ* by direct injection or by use of an infusion pump.

### The main advantages of the present invention include:

1. The TF nanobody of the present invention has excellent biological activity, specificity, and high affinity. The binding affinity constant (KD) values of chimeric nanobodies 4-A02, F1-A01, 4-E04, 4-C05, 5-C12, and 5-H3 to human TF-ECD range from 0.202 to 7.04 nM, as determined by Surface Plasmon Resonance (SPR) analysis.
2. It has good binding affinity to TF on tumor cell, with an EC₅₀ of 0.05 µg/mL~0.75 µg/mL determined by FACS, and it can inhibit TF related functions in tumor cell, therefore be used as a targeted therapeutic antibody.
3. The nanobody-drug conjugate TF-NDC (microtubule inhibitor, topoisomerase 1 inhibitor) designed based on TF nanobodies can specifically kill tumor cells with medium and high expression of TF, with IC50 values ranging from 0.0001 µg/mL to 0.03 µg/mL.
4. The TF-NDC of the present invention exhibits significant anti-tumor activity, where a single administration of 3mg/kg and 1mg/kg demonstrating strong anti-tumor activity and potential for regulating the immune microenvironment *in vivo.*
5. According to the design of Figure 24 of the present invention, TF nanobody-drug conjugates with the required DAR values can be conveniently prepared, and their cross-linking reaction equations are shown in Figure 23 1a, 1b, 1c or 1d, respectively.

The present invention will be further illustrated below with reference to the specific examples. It should be understood that these examples are for illustrative purposes only and are not intended to limit the scope of the invention. For the experimental methods in the following examples, in which the specific conditions are not specifically indicated, they are performed under routine conditions, e.g., those described by Sambrook. et al., in Molecule Clone: A Laboratory Manual, New York: Cold Spring Harbor Laboratory Press, 1989, or as instructed by the manufacturers. Unless otherwise specified, percentages and parts are calculated by weight.

### Example 1 Discovery and Preparation of Nanobodies Targeting Human Tissue Factor (TF)

The human TF extracellular domain protein (TF-ECD, Met 1-Glu 251) was purchased from SinoBiological company (catalog number: 13133-H08H). The prepared TF extracellular domain protein was used to immunize Alpaca, with an interval of 21 days between immunizations. Partial peripheral blood cells (PBMCs) were collected 10 days after the last immunization. Yeast and phage display libraries were constructed after PBMC isolation and VHH antibody fragment cloning. Through steps such as repeated panning, monoclonal ELISA, and FACS screening, seven nanobodies (VHH) 4-A02, F1-A01, 4-C08, 4-E04, 4-C05, 5-C12, and 5-H3 with high activity and independent sequences were obtained from phage display library. Seven VHH amino acid sequences were fused with human Fc mutant fragment FC1 to prepare chimeric antibodies, which were then transiently transferred to HEK293 and suspended in serum-free medium for 6 days. The culture supernatant was collected for purification, protein quantification, and SDS-PAGE detection.

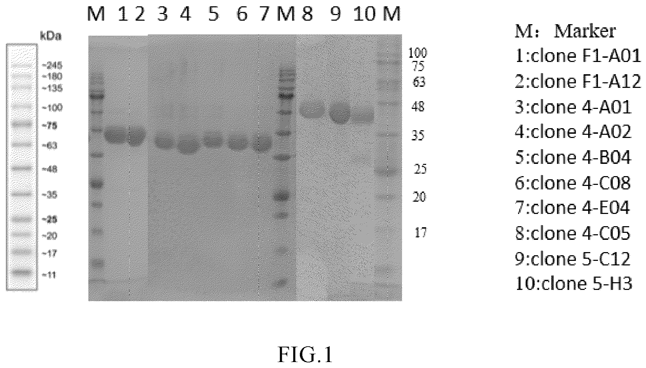

As shown in Figure 1, the fusion proteins derived from 4-A02, F1-A01, 4-C08, 4-E04, 4-C05, 5-C12, and 5-H3 were efficiently expressed, produced, and purified.

### Example 2 Detection of TF Nanobody Affinity to Human TF Antigen By SPR

Antibody affinity was detected using ForteBio OCTET R2 instrument. The NTA sensor (Sartorius, Cat#18-5101) was immobilized with FDYKSD-01-His for 200 s. The buffer was PBST (PBS + 0.02% Tween-20), and the nanobody samples were serially diluted to 25, 12.5, 6.25, 3.13, 1.56, and 0 nM. Affinity assay: baseline equilibration for 60 s, association for 180 s, and dissociation for 180 s at 25°C. Kinetic characterization was analyzed using the ForteBio OCTET R2 system.

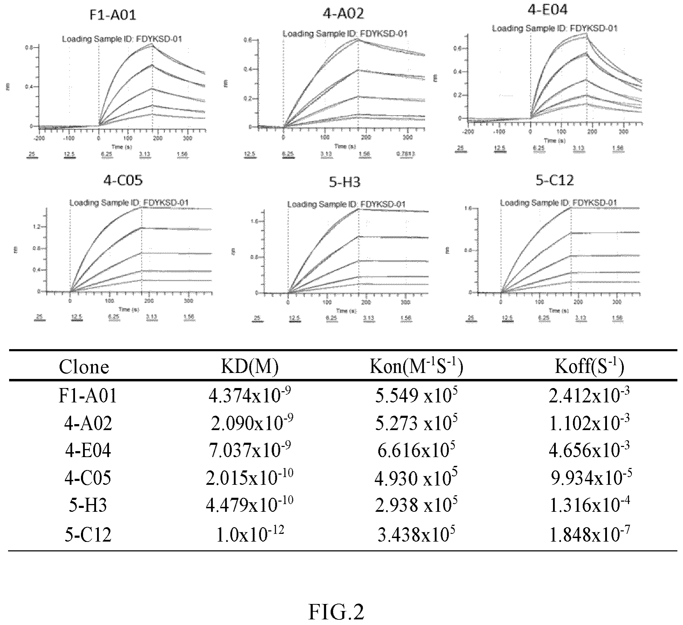

The detection results are shown in Figure 2. The chimeric antibodies 4-A02, F1-A01, 4-E04, 4-C05, 5-C12, and 5-H3 have strong affinity for TF-ECD, with binding constant (KD) values of 2.09nM, 4.37nM, 7.04nM, 0.2nM, 0.001nM, and 0.45nM, respectively.

### Example 3 Detection of Binding Affinity of TF Nanobody to TF on Tumor Cell Surface

TF-high-expressing triple-negative breast cancer cells MDA-MB-231 were used for the assay. 100 µL of test antibodies, which were serially diluted in a 5-fold gradient from 10 µg/mL to 0.016 µg/mL was set as primary antibodies and mixed with 1×10⁵ tumor cells suspended in 100 µL serum-free RPMI-1640 medium, resulting in the final antibody concentrations from 5 µg/mL to 0.008 µg/mL. After incubation at 4°C for 1 h, cells were washed twice with PBS to remove unbound primary antibodies. The target cells were then incubated with 200 µL of 2 µg/mL PE-labeled secondary antibody at 4°C for 30 min, and washed twice with PBS to remove unbound secondary antibodies. Finally, cells were resuspended in 200 µL PBS, and binding affinity of test antibodies to TF on cell surface was measured by flow cytometry.

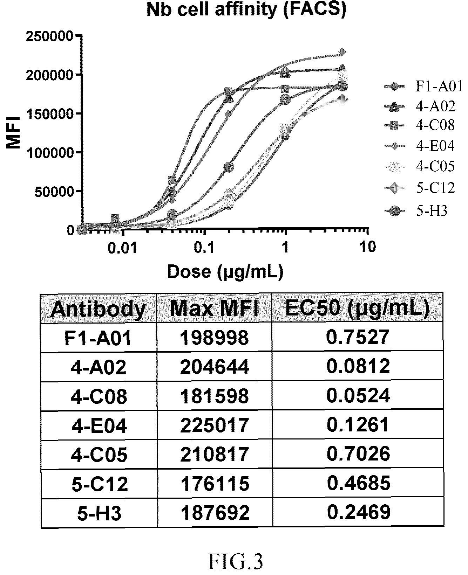

The detection results are shown in Figure 3. 4-A02, F1-A01, 4-C08, 4-E04, 4-C05, 5-C12, and 5-H3 have excellent binding affinity for MDA-MB-231, with EC₅₀ values of 0.08µg/mL, 0.75µg/mL, 0.05µg/mL, 0.13µg/mL, 0.7µg/mL, 0.47µg/mL, and 0.25µg/mL, respectively.

### Example 4 Preparation of TF Nanobody Fusion Protein-vc-MMAE

The protein stock solutions of FC1 chimeric naked antibodies (usually dimers) derived from 4-A02, F1-A01, 4-C08, 4-E04, 4-C05, 5-C12, and 5-H3 or 4-A02-ABD (monomer) were diluted with 50mM potassium dihydrogen phosphate-sodium hydroxide (KH₂PO₄-NaOH)/150mM sodium chloride (NaCl)/1mM diethylenetriaminepentaacetic acid (DTPA) reaction buffer (pH 7.4) to 5mg/mL, and added with 3-4-fold excess molar ratio of tris (2-carboxyethyl) phosphine hydrochloride (TCEP). The reaction solution was stirred at 25°C for 2.5 hours.

The above reaction solution was cooled to 0-10°C, and added with an appropriate amount of diethylacetamide (DMA) under an unpurified condition, then added with 6-fold excess molar ratio of mc-vc-MMAE (10mg/ml pre dissolved in DMA) to ensure that the volume proportion of DMA in the reaction system does not exceed 10%. The reaction solution was stirred at 10-25°C for 2 hours for coupling.

The coupling reaction mixture was filtered and purified by using a desalting column with pH 6.0 histidine-acetate/sucrose gel, and peak fractions were collected based on UV280 absorbance. The samples were then sterilized by filtration through a 0.22 µm pore-size filter device and stored at -80°C.

For the naked antibodies and MMAE conjugates, purity and coupling efficiency were analyzed by methods such as hydrophobic interaction chromatography (HIC) and size-exclusion chromatography (SEC). For dimeric antibody conjugates, the target DAR of payload was 4; for monomeric antibody-ABD conjugates, the target DAR was 1.

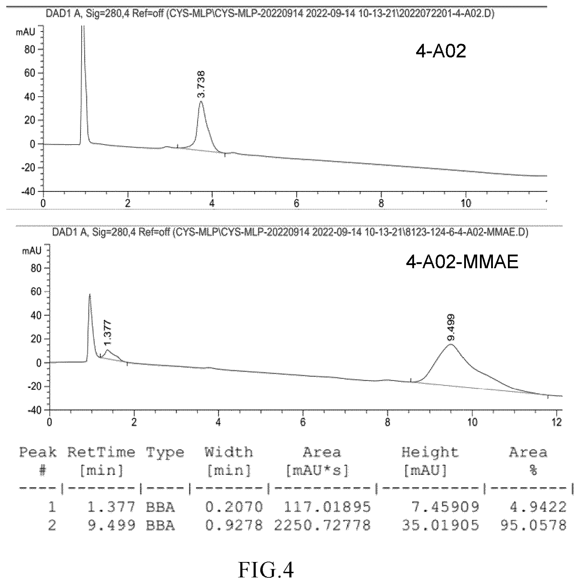

As shown in Figure 4, the proportion of DAR4 of 4-A02-MMAE conjugate detected by HIC was approximately 95%.

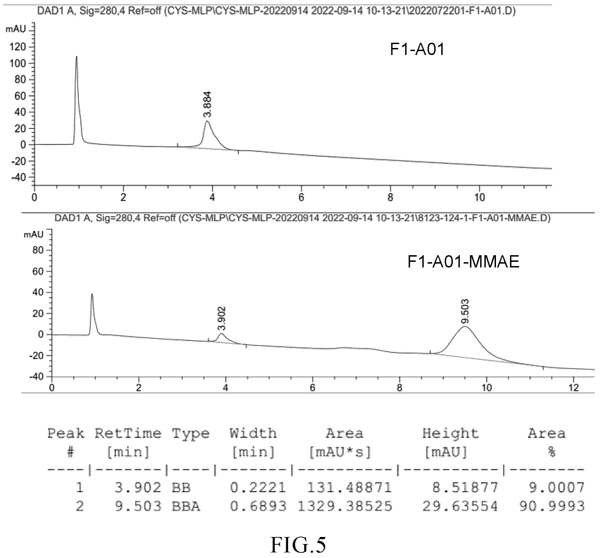

As shown in Figure 5, the proportion of DAR4 of F1-A01-MMAE conjugate detected by HIC was approximately 91%.

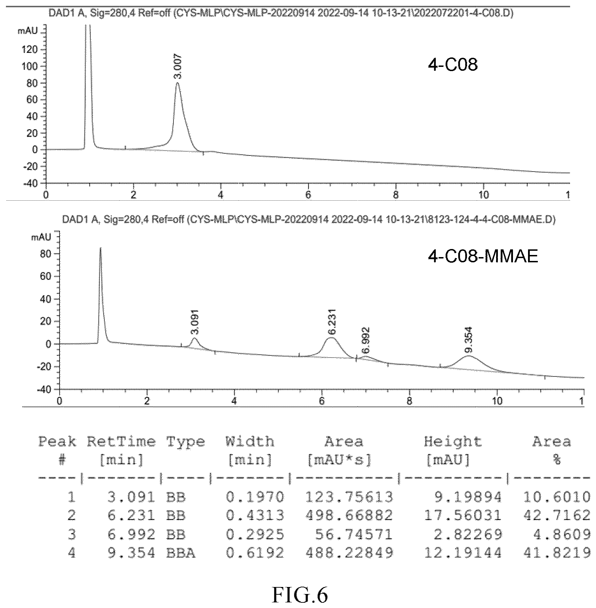

As shown in Figure 6, the proportions of DAR4 and DAR2 of 4-C08-MMAE conjugate detected by HIC were approximately 41.8% and 42.7%, respectively.

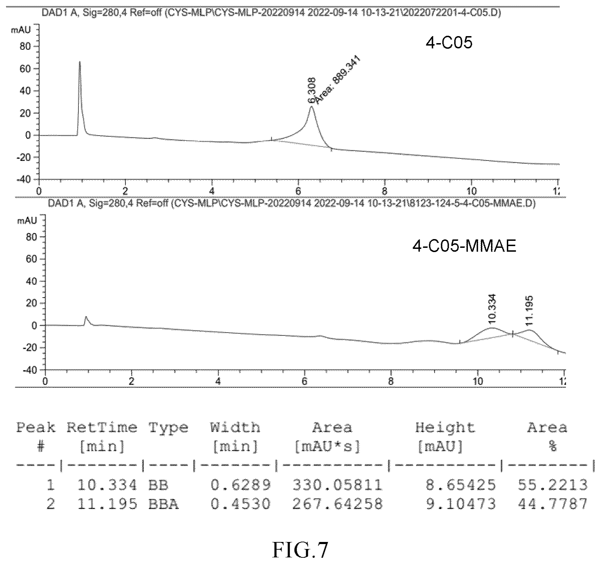

As shown in Figure 7, the HIC detection analysis of 4-C05-MMAE conjugate showed that the naked antibody had been largely converted into the conjugate.

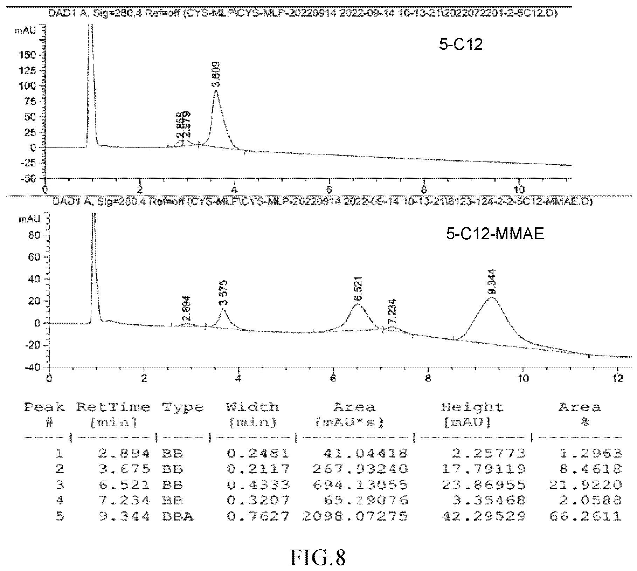

As shown in Figure 8, the proportions of DAR4 and DAR2 of 5-C12-MMAE conjugate detected by HIC were approximately 66.2% and 21.9%, respectively.

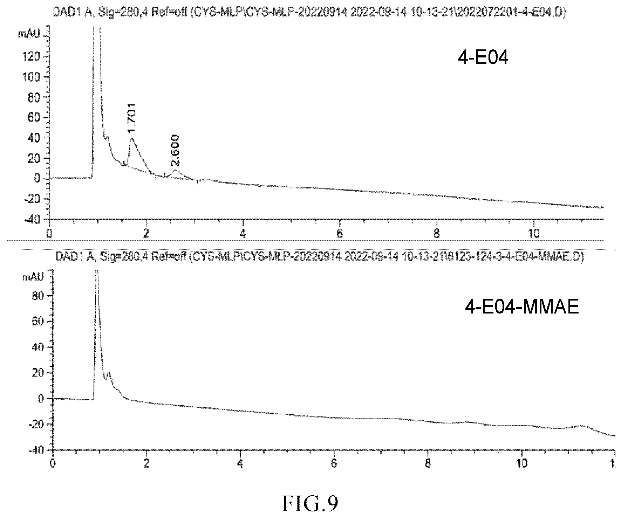

As shown in Figure 9, the HIC detection analysis of the 4-E04-MMAE conjugate showed that the coupling reaction did not complete as expected, and the proportion of coupling products was very low.

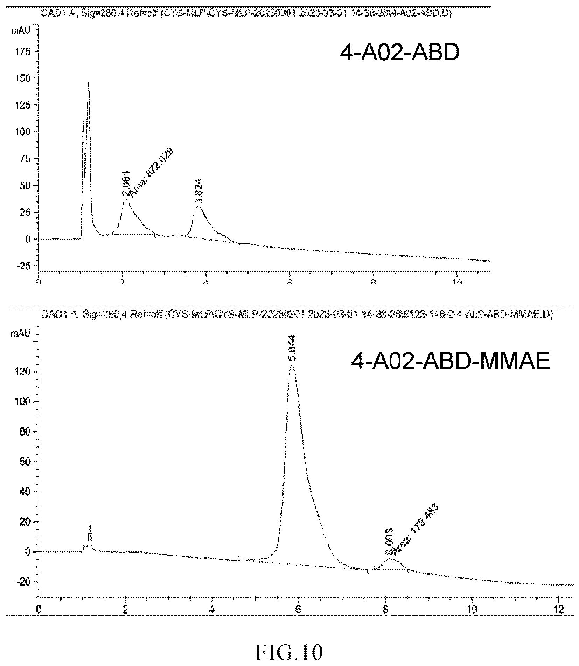

As shown in Figure 10, the HIC detection analysis of the 4-A02-ABD-MMAE conjugate showed that the naked antibody had been largely converted to the conjugate with DAR1.

### Example 5 Preparation of TF Nanobody-GGFG-DXd

The 4-A02-FC1 naked antibody solution was exchanged into a reaction buffer containing 50 mM sodium dihydrogen phosphate-disodium hydrogen phosphate (NaH₂PO₄-Na₂HPO₄)/150 mM sodium chloride (NaCl)/2 mM ethylene glycol tetraacetic acid (EDTA), with a pH of 7.0, and adjusted to a concentration of 10 mg/mL. Tris (2-carboxyethyl) phosphine hydrochloride (TCEP) was added at a 2.2-fold excess molar ratio, and the reaction solution was stirred at 25°C for 4-6 hours. The reaction solution was then cooled to room temperature, and an appropriate amount of dimethylacetamide (DMA) was added. Compound GGFG-DXd (purchased from Shanghai Haoyuan Chemical, pre-dissolved in DMA at 10 mg/mL) was added at a 4-fold excess molar ratio, ensuring the DMA volume percentage in the reaction system did not exceed 10%. The mixture was stirred at 25°C for 1-2 hours for coupling. Excess small molecules were removed using a G25 desalting column or ultrafiltration, and the solution was exchanged into a PBS/8% sucrose buffer with a pH of 7.4. The final product was sterilized by filtration through a 0.22 µm pore-size filter and stored at -80°C. The obtained antibody-drug conjugate was named as 4-A02-DXd.

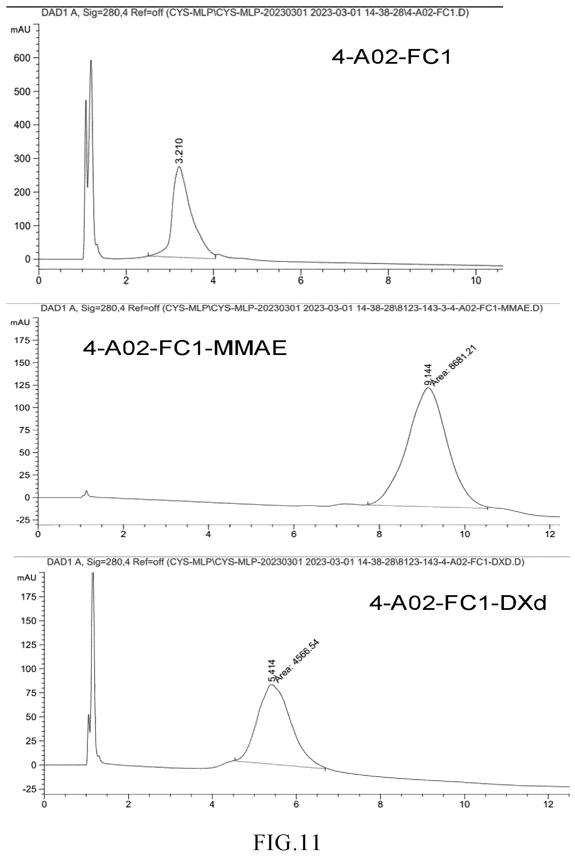

The results are shown in Figure 11. The HIC detection analysis of the 4-A02-DXd conjugate demonstrated complete conversion of the naked antibody to the conjugate with DAR4.

### Example 6: In Vitro Anti-tumor Activity of TF-NDC Against Tumor Cells with TF High/Medium Expression

The cell lines used in this example were purchased from the American Type Culture Collection (ATCC), Chinese Academy of Sciences Cell Bank, and Nanjing Kebai Biotechnology Co., Ltd., and cultured according to the corresponding instructions, including: triple-negative breast cancer cell lines HCC1806, MDA-MB-231, HCC1954, HCC1937, and MDA-MB-468; pancreatic cancer cell lines BxPC3, and HPAF-II; and non-small cell lung cancer cell lines HCC44, NCI-H1975, and PC9, etc.. Cells in logarithmic growth phase were inoculated into 96-well plates at densities of 200-2000 cells (depending on growth rates of different cells) in 150 µL medium/well and cultured at 37°C, 5% CO₂ for approximately 5 hours. Different concentrations of TF-NDCs (15 µg/mL to 0.00019 µg/mL) were then added, and 2-4 replicate wells for each drug concentration, along with vehicle control and blank control wells were set. After 5-9 days of culture (depending on cell growth rates to ensure sufficient times of cell division), the culture medium was removed and 100 µL/well of MTS reagent (Promega, cat# G3581) was added. The plates were subjected to incubation at 37°C until desired color was developed, then cell viability was determined by measuring OD490nm. Cell survival rate was calculated using the following formula: Survival rate = (OD treated - OD blank)/(OD control - OD blank) × 100%. Data were analyzed using GraphPad Prism 8 software to calculate IC₅₀ values for each TF-NDC to different cell lines.

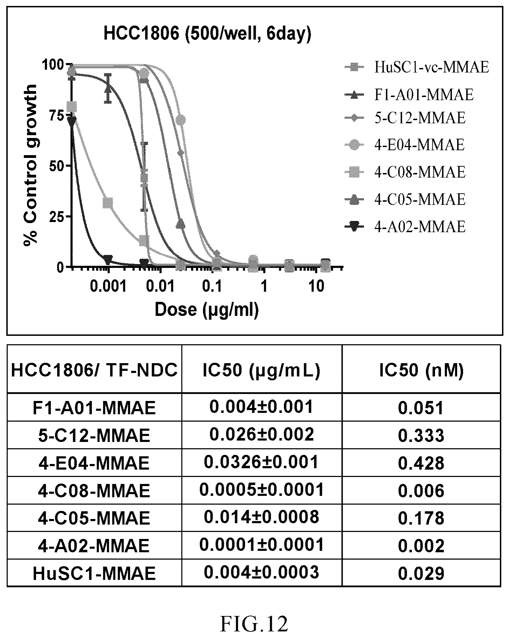

The *in vitro* anti-tumor efficacy results of the preferred TF-NDCs including 4-A02-MMAE, F1-A01-MMAE, 4-C08-MMAE, 4-C05-MMAE, 5-C12-MMAE, 4-E04-MMAE, and the control HuSC1-MMAE are shown in Figures 12, 13, and 14, respectively.

As shown in Figure 12, TF-NDCs exhibited potent proliferation inhibition activity on TF-high-expressing triple-negative breast cancer HCC1806 cells, with IC₅₀ values ranging from 0.0001 to 0.0326 µg/mL.

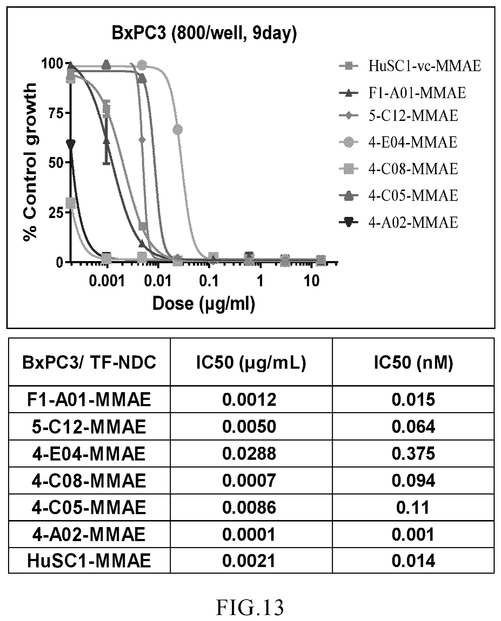

As shown in Figure 13, TF-NDCs exhibited potent proliferation inhibition activity on TF-high-expressing pancreatic cancer BxPC3 cells, with IC₅₀ values ranging from 0.0001 to 0.0288 µg/mL.

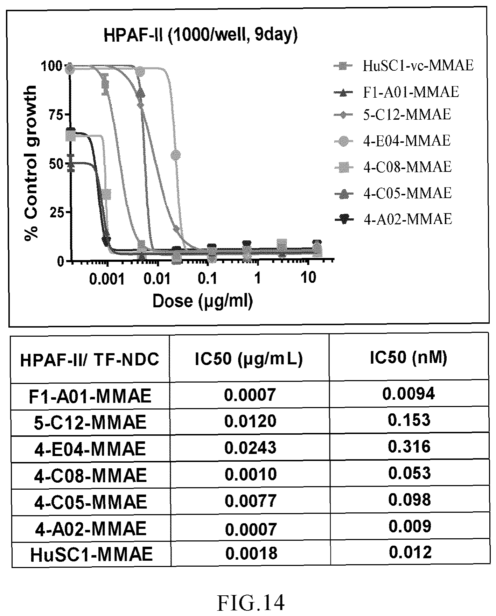

As shown in Figure 14, TF-NDCs exhibited potent proliferation inhibition activity on TF-high-expressing pancreatic cancer HPAF-II cells, with IC₅₀ values ranging from 0.0007 to 0.0243 µg/mL.

Table-3 summarizes the IC₅₀ values from cell proliferation inhibition assays for all six TF-NDCs.

**Table-3: In Vitro Anti-Tumor Activity of TF Nanobody-Drug Coniugates**

| TF-NDC Name | Cell proliferation inhibition IC₅₀ (µg/mL) | | |
|---|---|---|---|
| | HCC1806 | BxPC3 | HPAF-II |
| 4-A02-MMAE | 0.0001±0.0001 | 0.0001 | 0.0007 |
| F1-A01-MMAE | 0.004±0.001 | 0.0012 | 0.0007 |
| 4-C08-MMAE | 0.0005±0.0001 | 0.0007 | 0.0010 |
| 4-E04-MMAE | 0.0326±0.001 | 0.0288 | 0.0243 |
| 4-C05-MMAE | 0.014±0.0008 | 0.0086 | 0.0077 |
| 5-C12-MMAE | 0.026±0.002 | 0.0050 | 0.0120 |

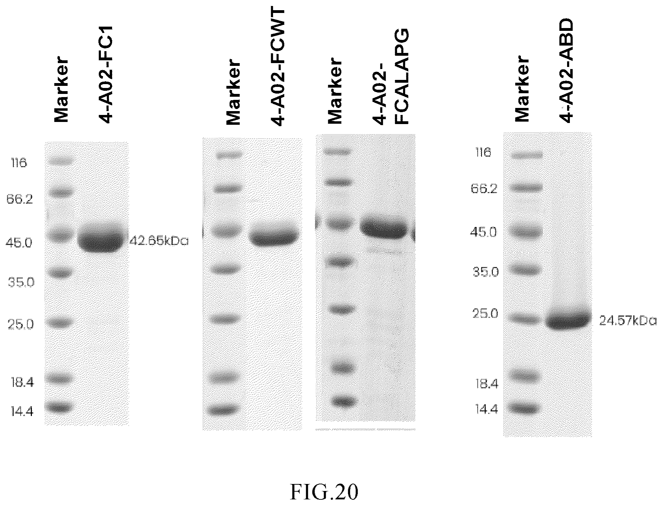

Based on the aforementioned anti-tumor activity assays, 4-A02 was selected for design and mass production, and 4-A02-FC1, 4-A02-FCWT, 4-A02-FCLALAPG, and 4-A02-ABD proteins were purified. The results are shown in Figure 20. Among them, 4-A02-FC1, 4-A02-FCWT, and 4-A02-FCLALAPG have a molecular weight of approximately 39 kDa; 4-A02-ABD protein has a molecular weight of approximately 20 kDa.

Using the above batch-purified proteins, as shown in Figures 10 and 11, 4-A02-MMAE was reproducibly prepared, and 4-A02-ABD-MMAE and 4-A02-DXd were designed and prepared.

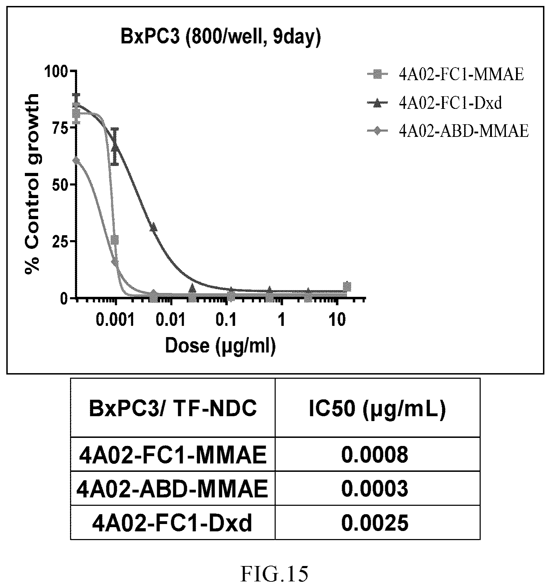

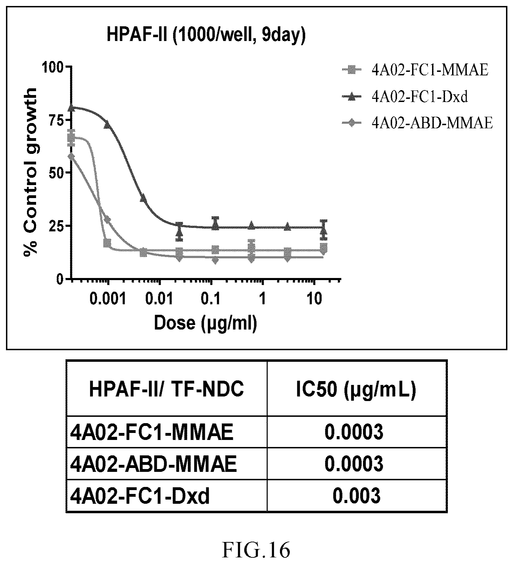

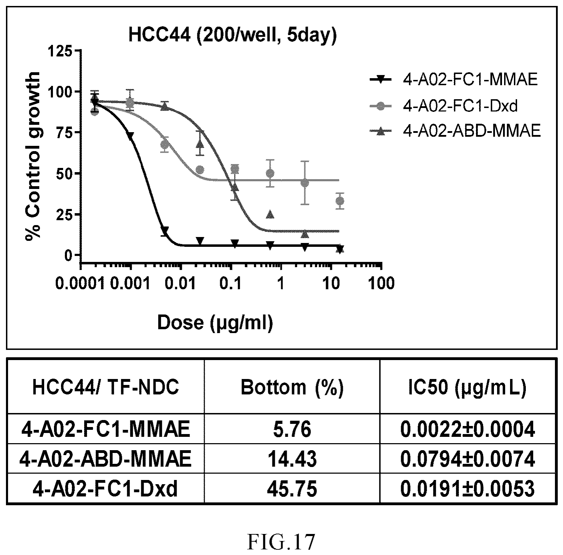

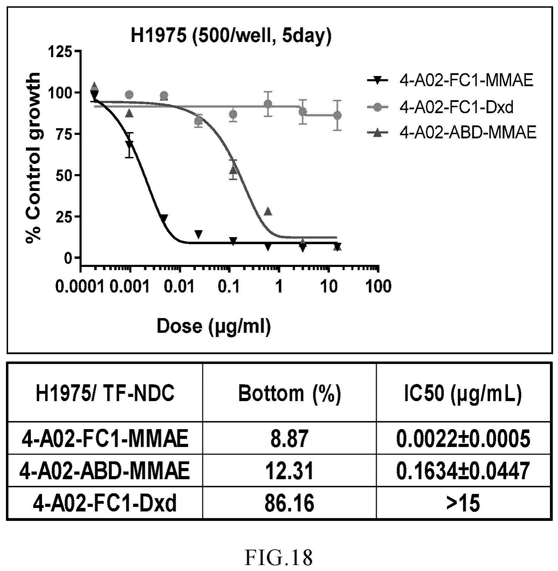

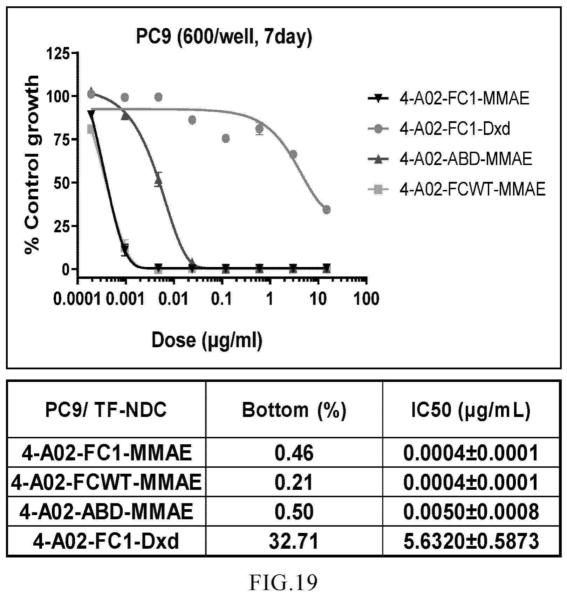

As shown in Figures 15, 16, 17, 18, and 19, both 4-A02-MMAE and 4-A02-ABD-MMAE exhibited potent proliferation inhibitory activity on HPAF-II, BxPC3, HCC44, PC9, and H1975 cells with high or medium TF expression, with IC₅₀ values ranging from 0.0002 to 0.022 µg/mL and 0.0003 to 0.163 µg/mL, respectively.

As shown in Figures 15, 16, 17, 18, and 19, 4-A02-DXd exhibited excellent proliferation inhibitory activity on HPAF-II, BxPC3, and HCC44 cells, with IC₅₀ values approximately ranging from 0.0024 to 0.019 µg/mL. The inhibitory activity of 4-A02-DXd on lung cancer PC9 and H1975 cells was relatively weaker, with IC₅₀ values of 5.63 µg/mL and > 15 µg/mL, respectively.

Table 4 summarizes the IC₅₀ values of proliferation inhibition assays for three TF-NDCs.

**Table 4: In Vitro Anti-Tumor Activity of TF Nanobody-Drug Conjugates**

| TF-NDC Name | Cell proliferation inhibition IC₅₀ (µg/mL) | | | | |
|---|---|---|---|---|---|
| | HPAF-II | HCC44 | PC9 | H1975 | BxPC3 |
| 4-A02-MMAE | 0.0002±0.0001 | 0.0022±0.0004 | 0.0004±0.0001 | 0.0022±0.0005 | 0.0008 |
| 4-A02-ABD-MMAE | 0.0013±0.0003 | 0.0794±0.0074 | 0.0050±0.0008 | 0.1634±0.0447 | 0.0003 |
| 4-A02-DXd | 0.0024±0.0008 | 0.0191±0.0053 | 5.6320±0.5873 | >15 | 0.0025 |

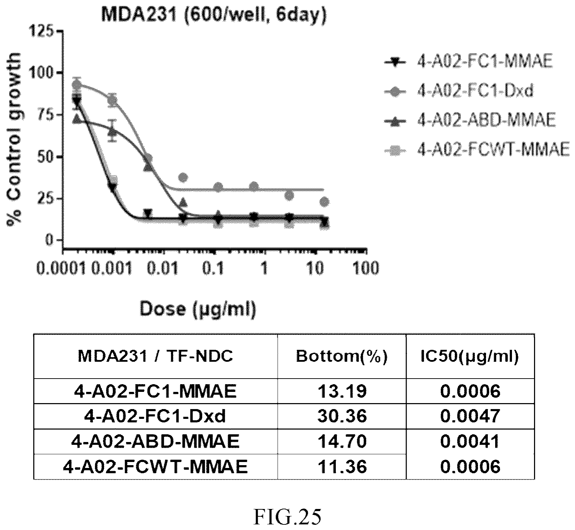

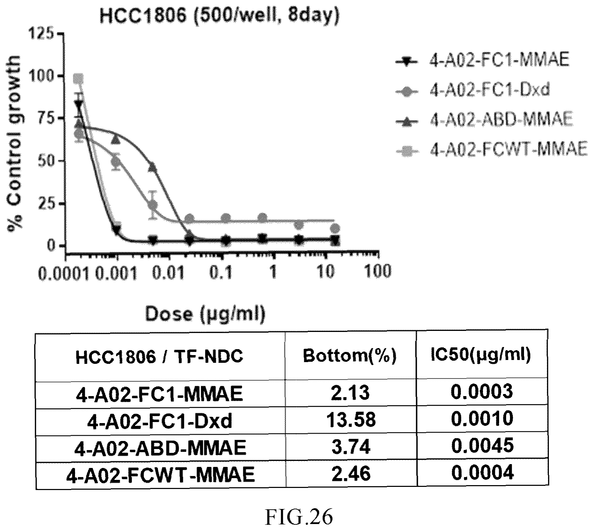

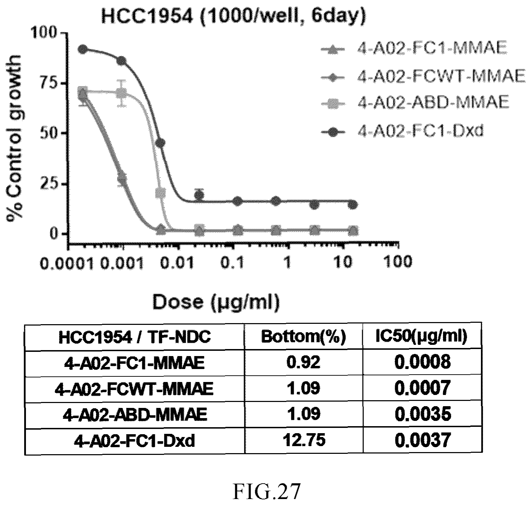

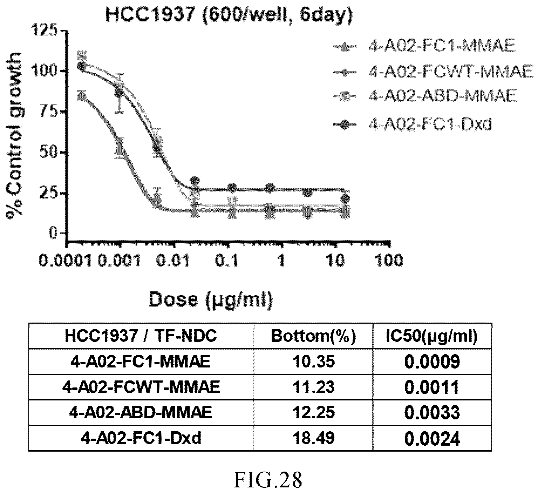

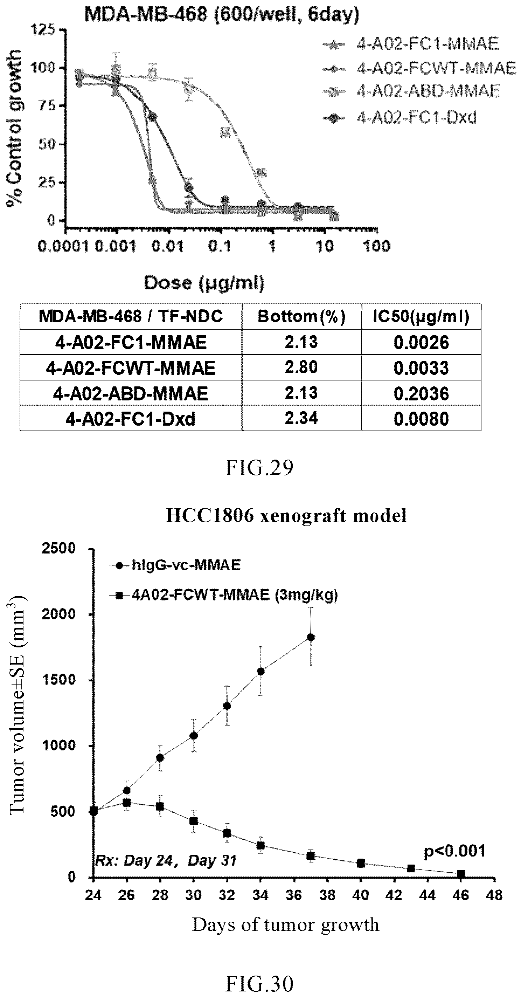

The *in vitro* anti-tumor efficacy of 4-A02-FC1-DXd, 4-A02-FC1-MMAE, 4-A02-FCWT-MMAE, and 4-A02-ABD-MMAE was further evaluated in multiple triple-negative breast cancer (TNBC) cell lines. As shown in Figure 25 (MDA-MB-231), Figure 26 (HCC1806), Figure 27 (HCC1954), Figure 28 (HCC1937), and Figure 29 (MDA-MB-468), 4-A02-DXd inhibited TNBC cell proliferation with IC₅₀ values ranging from 0.001 to 0.008 µg/mL. Similarly, the 4-A02-NDCs conjugated with MMAE also demonstrated potent anti-tumor effects, with IC₅₀ values ranging from 0.0003 to 0.004 µg/mL.

### Example 7 Batch Expression, Production, and Purification of TF Nanobodies and Fusion Proteins

The variable region sequences of the nanobodies were cloned into FC1, FCWT, FCLALAPG, and ABD expression vectors (4-A02-FC1, 4-A02-FCWT, 4-A02-FCLALAPG, 4-A02-ABD). After transient transfection of the vectors into HEK293 cells, the cells were cultured in serum-free suspension medium for 6 days. The culture supernatants were collected for purification, protein quantification, and SDS-PAGE assay.

As shown in Figure 20, the chimeric nanobodies and recombinant fusion proteins derived from 4-A02 were efficiently expressed, produced, and purified.

### Example 8 In Vivo Anti-Tumor Effects of TF Nanobodies (Triple-negative Breast Cancer Mammary Pad Xenograft Model)

Triple-negative breast cancer (TNBC) HCC1806 cells in logarithmic growth phase were inoculated into the mammary pads of 6-week-old female Balb/c nude mice (purchased from Shanghai Sippr-BK Laboratory Animal Co., Ltd.) at a density of 3×10⁶ cells per 200 µL of serum-free medium. When the size of tumors reached 100-200 mm³, the animals were randomly divided into groups, with 8 tumors per group. The six preferred TF-NDCs of the present invention were administered via tail vein injection at a dose of 3 mg/kg on the day of grouping, and hIgG1-MMAE was used as the negative control drug. Only one dose was administered throughout the experiment. Tumor volume and nude mouse weight were measured and recorded 2-3 times per week to plot tumor growth curves. Tumor volume (V) was calculated using the formula: V = 1/2 × a × b², where a and b represent the length and width of the tumor, respectively.

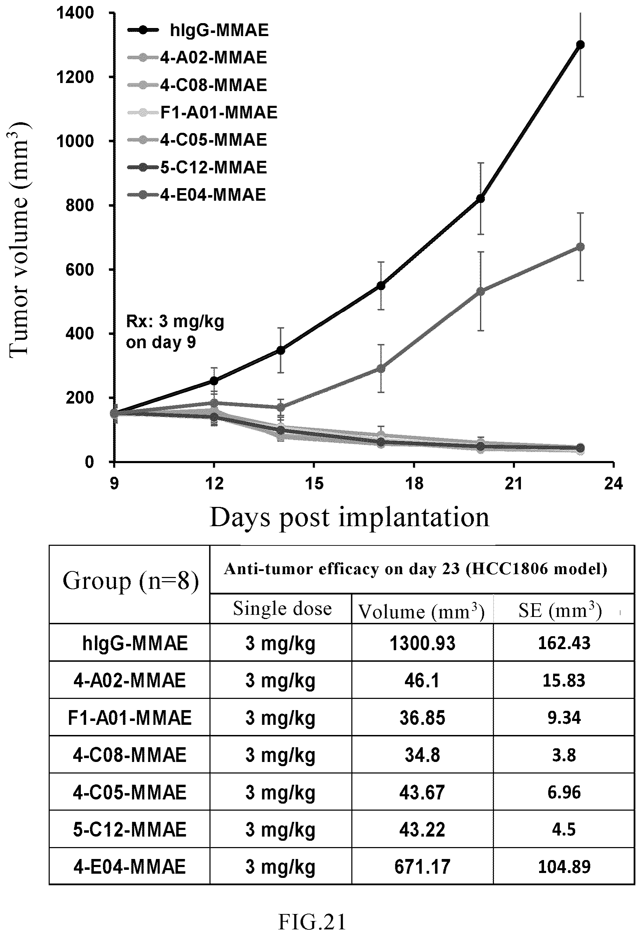

As shown in Figure 21, compared with the hIgG1-MMAE control group, the 3 mg/kg dose groups of 4-A02-MMAE, F1-A01-MMAE, 4-C08-MMAE, 4-C05-MMAE, and 5-C12-MMAE all demonstrated potent anti-tumor activity, resulting in tumor regression. In addition, the conjugate 4-E04-MMAE only showed partial tumor inhibition, which may be related to its lower coupling efficiency (Figure 9).

The *in vivo* anti-tumor efficacy of 4-A02-FC1-DXd, 4-A02-FC1-MMAE, 4-A02-FCWT-MMAE, and 4-A02-ABD-MMAE was further evalued in triple-negative breast cancer models.

As shown in Figure 30, in the HCC1806 tumor regression experiment, the 3 mg/kg dose of 4-A02-MMAE group resulted in complete regression of large tumors within 21 days.

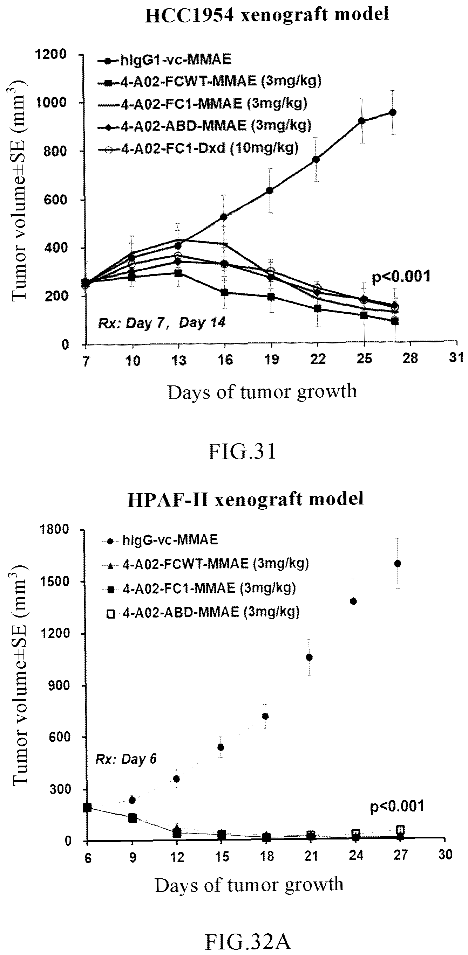

As shown in Figure 31, in the HCC1954 mammary pad xenograft experiment, 10 mg/kg 4-A02-FC1-DXd, 3 mg/kg 4-A02-MMAE, and 3 mg/kg 4-A02-ABD-MMAE all demonstrated significant inhibition of tumor growth and caused tumor regression.

### Example 9 In vivo anti-tumor effects of TF nanobodies (lung cancer orthotopic xenograft model)

4-5 week old nude mice were purchased and cultured for one week in SPF-level animal laboratory. HCC44-luc cells in logarithmic growth phase were digested and centrifuged using a 4°C pre-cooled centrifuge. Cells were resuspended in PBS according to cell count and adjusted to a density of 40×10⁶/mL, then an equal volume of high-concentration Matrigel was added, i.e., cell suspension:Matrigel=1:1. After anesthetizing mice with avertin (400 µL/20g), a small incision was made on the right side of the rib cage near the armpit (through three layers of skin and muscle) to expose the pink lung tissue, avoiding the Y-shaped blood vessels on the skin. The needle was inserted at the center of the Y-shape. A line was marked at 3mm from the insulin needle tip to indicate injection depth. Cells were slowly injected (50 µL per mouse, total of 1×10⁶ cells/mouse). After injection, the needle was held for 5-10 seconds before being rotated out, and the wound was sutured. On day 13, mice were imaged using an *in vivo* imaging system and grouped based on signal values. On day 14, 3 mg/kg hIgG1-MMAE or 4-A02-MMAE was administered via tail vein injection (n=6-8). Only one dose was administered throughout the experiment. Fluorescence values were measured and recorded using the *in vivo* imaging system on days 20, 27 and 34.

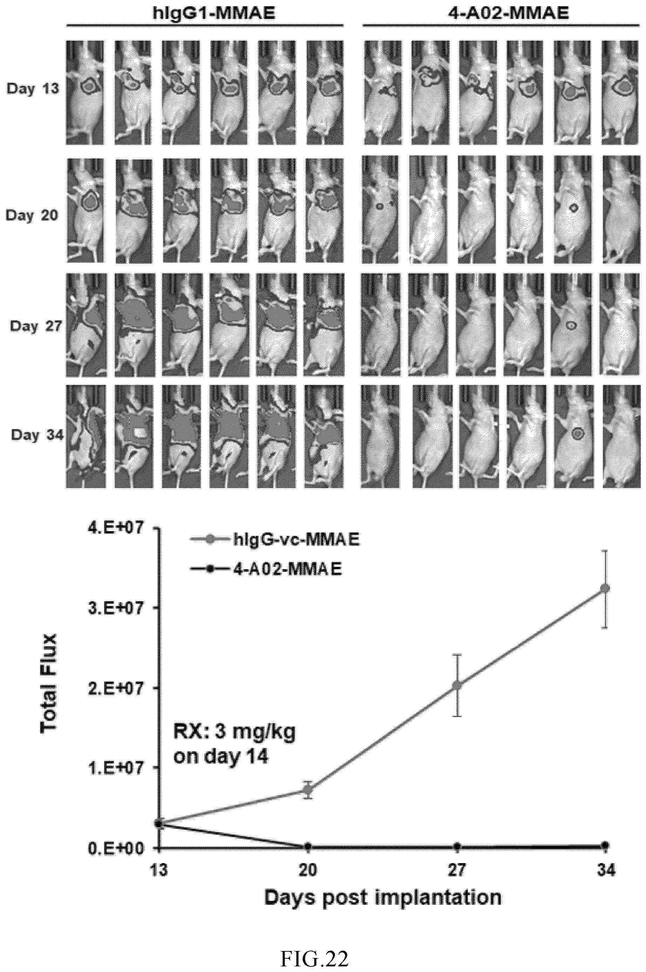

As shown in Figure 22, compared with the hIgG1-MMAE control group, the 3 mg/kg dose group of 4-A02-MMAE showed excellent anti-tumor activity, resulting in complete tumor regression in 5/6 mice.

### Example 10 In Vivo Anti-Tumor Effects of TF Nanobodies (Pancreatic Cancer Xenograft Model)

Pancreatic ductal adenocarcinoma (PDAC) HPAF-II cells in logarithmic growth phase were subcutaneously inoculated into the bank of 5-week-old female Balb/c nude mice (purchased from Shanghai Sippr-BK Laboratory Animal Co., Ltd.) at a density of 2.5×10⁶ cells per 200 µL serum-free medium. When tumor volume reached 100-200 mm³, animals were randomly grouped, 8 tumors per group. The preferred TF-NDCs such as 4-A02-MMAE, 4-A02-ABD-MMAE were administered via tail vein injection at 3 mg/kg or 1 mg/kg on the day of grouping, with hIgG1-MMAE as the negative control. Only one dose was administered throughout the experiment. Tumor volume and mouse body weight were measured twice a week to plot growth curves. Tumor volume (V) was calculated using the formula: V = 1/2 × a × b², where a and b represent tumor length and width, respectively.

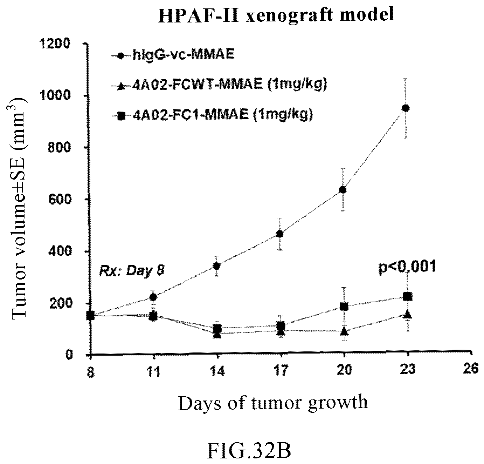

As shown in Figure 32A, compared with the hIgG1 group, single-dose administration of 3 mg/kg 4-A02-FC1-MMAE, 4-A02-FCWT-MMAE, or 4-A02-ABD-MMAE achieved complete tumor regression. As shown in Figure 32B, compared with the hIgG1 group, single-dose administration of 1mg/kg 4-A02-FC1-MMAE and 4-A02-FCWT-MMAE demonstrated excellent anti-tumor activity, achieving complete growth inhibition and partial tumor regression.

### Example 11 Humanization and Activity Detection of TF Nanobodies

Based on the original antibody sequence, a homology model was constructed to analyze framework amino acids within the 5Å range of the CDRs. These amino acid residues may influence CDR conformation or antigen-binding activity. Human germline was identified via IMGT analysis, and the selected human germline frameworks were grafted with the antibody CDRs to compare the designed humanized antibody frameworks with the original sequences. By analyzing the parental antibody's homology model, surface residues were substituted with amino acids similar to surface residues of human antibodies to minimize heterology while preserving activity, and humanized nanobodies 4A02-HM7, 4A02-HM8, and 4A02-HM9 were obtained, with sequences corresponding to SEQ ID NO.33, SEQ ID NO.34, and SEQ ID NO.35, respectively.

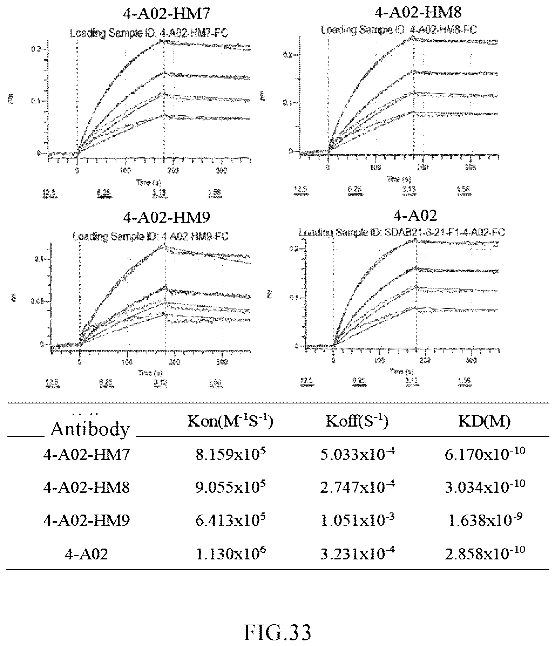

The binding affinity of humanized nanobodies to the antigen was determined using the surface plasmon resonance (SPR) assay method described in Example 2. As shown in Figure 33, 4A02-HM7, 4A02-HM8, and 4A02-HM9 exhibited nearly identical antigen-binding affinity to the original nanobody 4A02. In the same assay, the binding constants were 0.62 nM, 0.3 nM, 1.64 nM, and 0.29 nM, respectively.

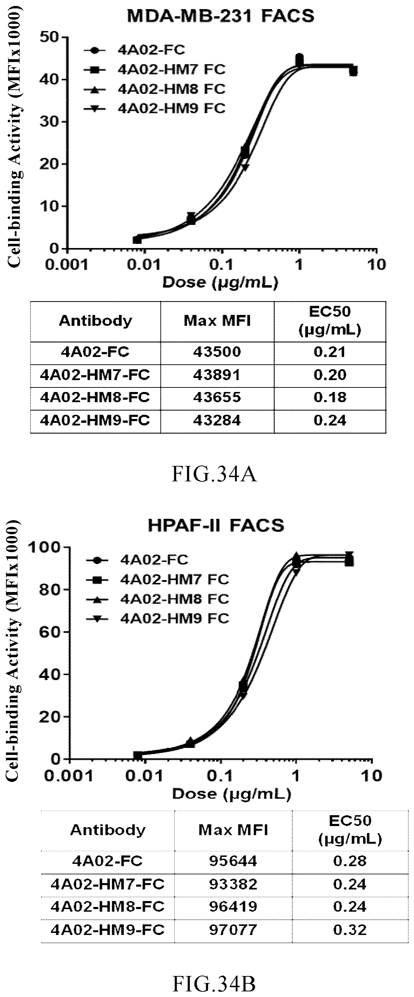

The cell-binding activity of humanized antibodies was evaluated using the tumor cell surface TF binding affinity assay method described in Example 3. As shown in Figure 34A, the binding affinity EC₅₀ of 4A02-HM7, 4A02-HM8, 4A02-HM9, and the original nanobody 4A02 to triple-negative breast cancer MDA-MB-231 cells ranged from 0.18 to 0.21 µg/mL. As shown in Figure 34B, the binding affinity EC₅₀ of 4A02-HM7, 4A02-HM8, 4A02-HM9, and the original nanobody 4A02 to HPAF-II cells ranged from 0.24 and 0.32 µg/mL.

### Example 12 ELISA Detection of the Affinity of Humanized TF Nanobody to Human TF and Cynomolgus Monkey TF Antigens

The extracellular domain fragments of TF proteins (human TF-ECD, cynomolgus monkey TF-ECD) were diluted to 1 µg/mL in coating buffer and used to coat ELISA plates (100 µL/well) at 4°C overnight. After washing away excess antigens, plates were blocked with 1% BSA at room temperature for 2 hours, then 5-fold gradient diluted nanobodies were added at 100 µL/well and incubated at room temperature for 1 hour. Unbound antibodies were washed away, and horseradish peroxidase-conjugated anti-Fc secondary antibody in an appropriate concentration was added at 100 µL/well. The plates were incubated at room temperature, then unbound secondary antibody was washed away, and TMB substrate was added for color development for 15 minutes. 1M HCl (50 µL/well) was added to terminate color development reaction, then absorbance was measured at 450 nm for data analysis.

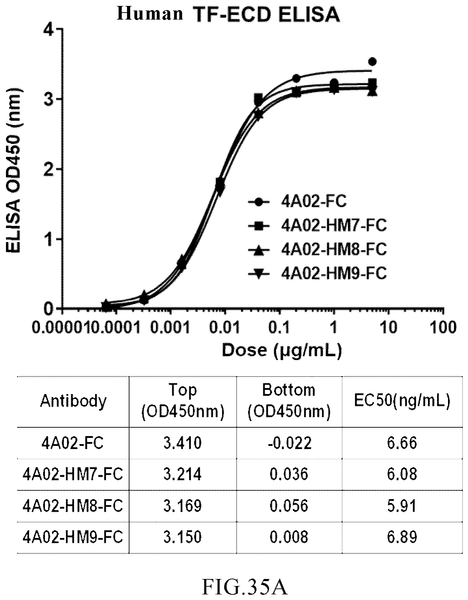

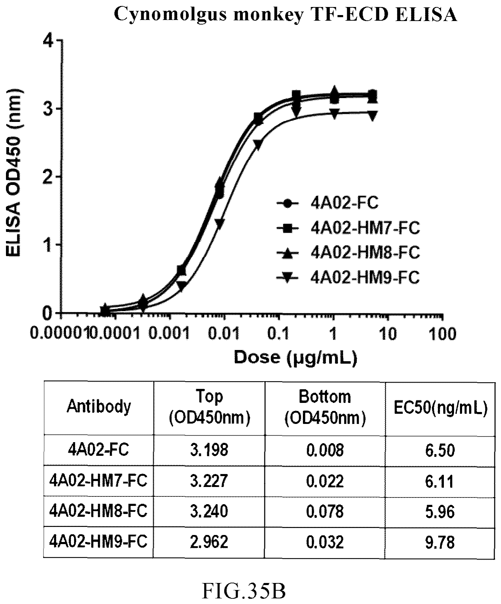

The ELISA results showed that the binding affinity EC₅₀ of 4A02-HM7, 4A02-HM8, 4A02-HM9, and the original nanobody 4A02 to human TF-ECD ranged from 5.91 to 6.89ng/mL (Figure 35A), and the binding affinity EC₅₀ for cynomolgus monkey TF-ECD ranged from 5.96 to 9.78ng/mL (Figure 35B).

In summary, the pharmacodynamic studies in TF-NDC related examples clearly demonstrate:
1. TF-NDC 4-A02-MMAE, F1-A01-MMAE, 4-C08-MMAE, 4-C05-MMAE, and 5-C12-MMAE all exhibit excellent TF-specific tumor cell killing activity, showing strong proliferation inhibitory effects on tumor cells with medium or high TF expression, while having no significant toxicity to cells with low TF expression;
2. The 4-A02-ABD-MMAE prepared based on 4-A02-ABD also exhibits excellent anti-tumor activity;
3. 4-A02-DXd shows extremely strong or good anti-tumor activity *in vitro* in preferred pancreatic cancer, multiple triple-negative breast cancer cells and lung cancer cells;
4. In triple-negative breast cancer, lung cancer and pancreatic cancer *in vivo* pharmacodynamic model, the preferred TF-NDCs of the present invention all demonstrates excellent anti-tumor therapeutic effect after a single-dose intravenous administration.

The sequence of nanobodies of the present invention

| | | |
|---|---|---|
| SEQ ID NO.1: | 4-A02 CDR1 | ETISSTYI |
| SEQ ID NO.2: | 4-A02 CDR2 | ISGDGVTH |
| SEQ ID NO.3: | 4-A02 CDR3 | YAAGRWNH |
| SEQ ID NO.4: | 4-A02 VH | |
| QVQLVESGGGLVQPGGSLSLSCTASETISSTYIMGWHRRSPGKERELVAVISGDGVTHYADF VKGRFVISRDNAKNAVYLKMNFLTPEDTAVYYCYAAGRWNHWGQGTQVTVSS | | |
| SEQ ID NO.5: | F1-A01 CDR1 | GRAFSAYA |
| SEQ ID NO.6: | F1-A01 CDR2 | ISWSGGST |
| SEQ ID NO.7: | F1-A01 CDR3 | NADSLLSLLDGSRGGPGTDSGS |
| SEQ ID NO.8: | F1-A01 VH | |
| EVQLVESGGGLVQAGGSLRLSCAASGRAFSAYAMGWFRQAPGKEREFVAAISWSGGSTYY ADSVRGRFTISRDNTKNTVYLQMNSLKPEDTAVYYCNADSLLSLLDGSRGGPGTDSGSWGQGT QVTVSS | | |
| SEQ ID NO.9: | 4-C08 CDR1 | GFSLSSYD |
| SEQ ID NO.10: | 4-C08 CDR2 | IHSSGGYP |
| SEQ ID NO.11: | 4-C08 CDR3 | NLPPSRRWYKDY |
| ID NO.12: SEQ ID NO.12: 4-C08 VH | 4-C08 VH | |
| DVQLVESGGGLVQPGGSLRLSCAVSGFSLSSYDMNWYRQGPGKEREWVANIHSSGGYPDY ADAVKGRFTISRDNAKNMVYLQMNSLKPEDTAVYYCNLPPSRRWYKDYWGQGTQVTVSS | | |
| SEQ ID NO.13: | 4-E04 CDR1 | EMISSTYI |
| SEQ ID NO.14: | 4-E04 CDR2 | ISGDGVTH |
| SEQ ID NO.15: | 4-E04 CDR3 | NAAGRRNH |
| SEO ID NO.16: | 4-E04 VH | |
| AVQLVESGGGLVQPGGSLSLSCAASEMISSTYIMGWYRRSPGKERELVAVISGDGVTHYGD FVKGRFAISRDNVKNAVYLKMNFLTPEDTAVYYCNAAGRRNHWGQGTQVTVSS | | |
| SEQ ID NO.17: | 4-C05 CDR1 | GFTLDTYA |
| SEQ ID NO.18: | 4-C05 CDR2 | ISSTDGST |
| SEQ ID NO.19: | 4-C05 CDR3 | AAGPGTDCPLRFDY |
| SEQ ID NO.20: SEQ ID NO.20 4-C05 | 4-C05 VH | |
| QVQLVESGGGLVQPGGSLRLSCAASGFTLDTYAIGWIRQAPGKEREGVSCISSTDGSTYYAA SVKGRFTINRDNAKNTVYLQMNSLKPEDTAVYYCAAGPGTDCPLRFDYWGQGIQVTVSS | | |
| SEQ ID NO.21: | 5-C12 CDR1 | GRTFSTDA |
| SEQ ID NO.22: | 5-C12 CDR2 | INWSGGST |
| SEQ ID NO.23: | 5-C12 CDR3 | VADSLLALLDGSRGGPGTDSDS |
| SEQ ID NO.24: | 5-C12 VH | |
| QVQLVESGGGLAQAGGSLRLSCAGSGRTFSTDAMGWFRQAPGKEREFVAAINWSGGSTY YADSVKGRFTISRDNAKNTVYLQMNSVKPEDTAVYYCVADSLLALLDGSRGGPGTDSDSWGQ GTQVTVSS | | |
| SEQ ID NO.25: | 5-H3 CDR1 | GFTLANYA |
| SEQ ID NO.26: | 5-H3 CDR2 | ISRSDGDT |
| SEQ ID NO.27: | 5-H3 CDR3 | RATEWCGVQDPHGY |
| SEQ ID NO.28: | 5-H3 VH | |
| QVQLVESGGGLVQPGGSLRLSCVASGFTLANYAIGFYRRAPGKGREGVSCISRSDGDTYH VDSVKGRFTVSRDNAKNTAYLQMNSLVPEDTGVYYCRATEWCGVQDPHGYWGQGTQVTVSS | | |
| SEQ ID NO.29: | 4-A02-FC1 | |
| QVQLVESGGGLVQPGGSLSLSCTASETISSTYIMGWHRRSPGKERELVAVISGDGVTHYAD FVKGRFVISRDNAKNAVYLKMNFLTPEDTAVYYCYAAGRWNHWGQGTQVTVSSEPKSSDKT HTCPPCPAPEFEGGPSVFLFPPKPKDTLMISRTPEVTCVVVDVSHEDPEVKFNWYVDGVEVHNA | | |
| KTKPREEQYNSTYRVVSVLTVLHQDWLNGKEYKCKVSNKALPASIEKTISKAKGQPREPQVYT LPPSRDELTKNQVSLTCLVKGFYPSDIAVEWESNGQPENNYKTTPPVLDSDGSFFLYSKLTVDK SRWQQGNVFSCSVMHEALHNHYTQKSLSLSPGK | | |
| SEQ ID NO.30: | 4-A02-FCWT | |
| QVQLVESGGGLVQPGGSLSLSCTASETISSTYIMGWHRRSPGKERELVAVISGDGVTHYAD FVKGRFVISRDNAKNAVYLKMNFLTPEDTAVYYCYAAGRWNHWGQGTQVTVSSEPKSSDKT HTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYT LPPSREEMTKNQVSLTCLVKGFYPSDIAVEWESNGQPENNYKTTPPVLDSDGSFFLYSKLTVDK SRWQQGNVFSCSVMHEALHNHYTQKSLSLSPGK | | |
| SEQ ID NO.31: | 4-A02-FCLALAPG | |
| QVQLVESGGGLVQPGGSLSLSCTASETISSTYIMGWHRRSPGKERELVAVISGDGVTHYADFVKGRF VISRDNAKNAVYLKMNFLTPEDTAVYYCYAAGRWNHWGQGTQVTVSSEPKSSDKTHTCPPCPAPEAAG GPSVFLFPPKPKDTLMISRTPEVTCVVVDVSHEDPEVKFNWYVDGVEVHNAKTKPREEQYNSTYRVVSV LTVLHQDWLNGKEYKCKVSNKALGAPIEKTISKAKGQPREPQVYTLPPSREEMTKNQVSLTCLVKGFYPS DIAVEWESNGQPENNYKTTPPVLDSDGSFFLYSKLTVDKSRWQQGNVFSCSVMHEALHNHYTQKSLSLS PGK | | |
| SEQ ID NO.32: | 4-A02-ABD | |
| QVQLVESGGGLVQPGGSLSLSCTASETISSTYIMGWHRRSPGKERELVAVISGDGVTHYAD FVKGRFVISRDNAKNAVYLKMNFLTPEDTAVYYCYAAGRWNHWGQGTQVTVSSGGGGSGGG GSGGGGSQHDEAVDANSLAEAKVLANRELDKYGVSDYYKNLINNAKTVEGVKALIDEILAALP AAACAAAHHHHHH | | |
| SEQ ID NO.33: | 4A02-HM7-FCWT | |
| QVQLVESGGGLVQPGGSLRLSCTASETISSTYIMGWHRQAPGKGRELVAVISGDGVTHYA DFVKGRFTISRDNAKNTVYLQMNFLRAEDTAVYYCYAAGRWNHWGQGTMVTVSSEPKSSDK THTCPPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQVY TLPPSREEMTKNQVSLTCLVKGFYPSDIAVEWESNGQPENNYKTTPPVLDSDGSFFLYSKLTVD KSRWQQGNVFSCSVMHEALHNHYTQKSLSLSPGK | | |
| SEQ ID NO.34: | 4A02-HM8-FCWT | |
| QVQLVESGGGLVKPGGSLRLSCTASETISSTYIMGWHRQAPGKGRELVAVISGDGVTHYA DFVKGRFTISRDNAKNTVYLQMNFLRAEDTAVYYCYAAGRWNHWGQGTMVTVSSEPKSSDK THTCPPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQVY TLPPSREEMTKNQVSLTCLVKGFYPSDIAVEWESNGQPENNYKTTPPVLDSDGSFFLYSKLTVD KSRWQQGNVFSCSVMHEALHNHYTQKSLSLSPGK | | |
| SEQ ID NO.35: | 4A02-HM9-FCWT | |
| QVQLVESGGGLVQPGGSLRLSCTASETISSTYIMGWHRQAPGKGLELVAVISGDGVTHYA DFVKGRFTISRDNAKNAVYLQMNFLRAEDTAVYYCYAAGRWNHWGQGTMVTVSSEPKSSDK THTCPPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQVY TLPPSREEMTKNQVSLTCLVKGFYPSDIAVEWESNGQPENNYKTTPPVLDSDGSFFLYSKLTVD KSRWQQGNVFSCSVMHEALHNHYTQKSLSLSPGK | | |

All literatures mentioned in the present application are incorporated herein by reference, as each one is individually incorporated by reference. In addition, it should also be understood that, after reading the above teachings of the present invention, those skilled in the art can make various changes or modifications, and these equivalents also fall in the scope of claims as defined in the appended claims.

## Claims

1. A nanobody targeting tissue factor (TF), wherein the complementary determining region (CDR) of the VHH chain of the nanobody is one or more selected from the following groups:
(1) CDR1 as shown in SEQ ID NO.1,
CDR2 as shown in SEQ ID NO.2, and
CDR3 as shown in SEQ ID NO.3;
or,
(2) CDR1 as shown in SEQ ID NO.5,
CDR2 as shown in SEQ ID NO.6, and
CDR3 as shown in SEQ ID NO.7;
or,
(3) CDR1 as shown in SEQ ID NO.9,
CDR2 as shown in SEQ ID NO.10, and
CDR3 as shown in SEQ ID NO.11,
or,
(4) CDR1 as shown in SEQ ID NO.13,
CDR2 as shown in SEQ ID NO.14, and
CDR3 as shown in SEQ ID NO.15,
or,
(5) CDR1 as shown in SEQ ID NO.17,
CDR2 as shown in SEQ ID NO.18, and
CDR3 as shown in SEQ ID NO.19,
or,
(6) CDR1 as shown in SEQ ID NO.21,
CDR2 as shown in SEQ ID NO.22, and
CDR3 as shown in SEQ ID NO.23,
or,
(7) CDR1 as shown in SEQ ID NO.25,
CDR2 as shown in SEQ ID NO.26, and
CDR3 as shown in SEQ ID NO.27;
any one of the above amino acid sequences further comprises a derivative sequence that is optionally added, deleted, modified and/or substituted with at least one amino acid and may retain the binding affinity to TF

2. The nanobody of claim 1, wherein the VHH chain of the nanobody further comprises framework region (FR).

3. The nanobody of claim 1, wherein the VHH chain of the nanobody targeting TF has an amino acid sequence selected from the group consisting of: SEQ ID NO.4, SEQ ID NO.8, SEQ ID NO.12, SEQ ID NO.16, SEQ ID NO.20, SEQ ID NO.24, and SEQ ID NO.28; or the VHH chain of the nanobody targeting TF has an amino acid sequence as shown in positions 1-114 of SEQ ID NO.33, positions 1-114 of SEQ ID NO.34, or positions 1-114 of SEQ ID NO.35.

4. An antibody targeting TF, which comprises one or more VHH chains of the nanobody targeting tissue factor of any one of claims 1-3.

5. A multispecific antibody, which comprises: the nanobody targeting TF of any one of claims 1-3 or the antibody targeting TF of claim 4.

6. The multispecific antibody of claim 5, wherein the multispecific antibody further comprises a second antigen binding region targeting a target selected from the group consisting of: EGFR, TGFβ, BCMA, B7H6, GUCY2C, DLL3, CD38, CD123, CD19, CD20, CD22, B7-H3, GPC3, HER2, PMSA, CD28, 4-1BB, OX40, CD40, CD27, CD3, CTLA4, PD1, PDL1, CD73, BCMA, GLP-1, Trop2, TIGIT, LAG-3, FGL1, TLR7, and a combination thereof.

7. A recombinant protein comprising:
(i) a nanobody targeting TF, the nanobody targeting TF of any one of claims 1-3, the antibody targeting TF of claim 4, or the multispecific antibody of claim 5; and
(ii) an optional polypeptide molecule or fragment with therapeutic function; and/or
(iii) an optional functional domain for improvement of the physicochemical properties or druggability of protein.

8. The recombinant protein of claim 7, wherein the recombinant protein has the following structure from the N to C terminus or from the C to N terminus:
(A-B)m;
wherein, the element A is the nanobody targeting TF;
the element B is an Fc segment, an albumin binding domain (ABD) or an anti-albumin nanobody (HLE);
"-" represents a peptide bond or linker,
wherein m is a positive integer.

9. A CAR construct, wherein the antigen binding region of the CAR construct is the VHH chain of the nanobody of any one of claims 1-3.

10. A recombinant immune cell, wherein the immune cell expresses the exogenous CAR construct of claim 9.

11. An immunoconjugate comprising:
(a) an antibody moiety, wherein the antibody moiety is the nanobody targeting TF of any one of claims 1-3 or the antibody targeting TF of claim 4; and
(b) a coupling moiety coupled to the nanobody moiety, wherein the coupling moiety is selected from the group consisting of: a detectable label, a drug, a toxin, a cytokine, an enzyme, a protein degradation agent, an immune regulator (including immune agonist and inhibitor), an oligonucleotide, and a combination thereof.

12. The immunoconjugate of claim 11, wherein the immunoconjugate is the antibody-drug conjugate (ADC) as shown in the following molecular formula: 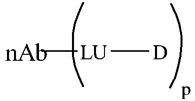 wherein:
nAb is the nanobody targeting TF or the recombinant protein of claim 5.
LU is linker 2 (also called a connector);
D is a drug;
and the subscript p is a value selected from 1-8.

13. The immunoconjugate of claim 12, wherein LU is a connector linked to the antibody moiety via a linker selected from the group consisting of maleimidocaproyl (MC), maleimide (MAL), and succinimidyl 4-(N-maleimidomethyl) cyclohexane-1-arboxylate) (SMCC), and comprises one or more selected from the group consisting of valine-citrulline (VC), valine-alanine (VA), glycine-glycine-phenylalanine-glycine (GGFG), alanine-alanine-alanine (AAA), p-aminobenzyloxycarbonyl (PAB), and polyethylene glycol (PEG).

14. The immunoconjugate of claim 12, wherein D is a compound with anti-tumor activity selected from the group consisting of:
(i) a tubulin inhibitor, such as maytansine derivative (DM1, DM4), monomethyl auristatin E (MMAE), and monomethyl auristatin F (MMAF);
(ii) a toxin that acts on DNA, such as duocarmycin and pyrrolobenzodiazepine (PBD);
(iii) a topoisomerase inhibitor, camptothecin, SN38, Exatecan, Dxd.

15. The immunoconjugate of claim 11, wherein the protein degradation agent is a degradation agent of a tumor-related protein selected from the group consisting of: EGFR, NF-κB, RIPK2, BCR-ABL, HER2, c-Met, TBK1, CDK, ALK, Akt, CK2, ERK1/2, FLT3, PI3K, BTK, TRK, Fak, BRD, AR, ER, MetAp-2, BCL-XL, Sirt2, HDAC6, Pirin, SMAD3, ARNT, PCAF/GCN5, Tau, EZH2, IRAK4, STAT3FRS2, and RAS (such as KRAS, HRAS and NRAS); preferably, the protein degradation agent comprises a PROTAC (proteolysis targeting chimera).

16. A pharmaceutical composition comprising:
(i) the nanobody targeting TF of any one of claims 1-3, the antibody targeting TF of claim 4, the multispecific antibody of claim 5, the recombinant protein of claim 7, the recombinant immune cell of claim 10, or the immunoconjugate of claim 11; and
(ii) a pharmaceutically acceptable carrier.

17. Use of an active ingredient, wherein the active ingredient is selected from the group consisting of: the nanobody targeting TF of any one of claims 1-3, the antibody targeting TF of claim 4, the multispecific antibody of claim 5, the recombinant protein of claim 7, the recombinant immune cell of claim 10, the immunoconjugate of claim 11, and a combination thereof, wherein the active ingredient is used for
(a) preparing a detection reagent, a detection plate or a kit for the detection of a TF-related disease; and/or
(b) preparing a medicine for the prevention and/or treatment of a TF-related disease.

18. The use of claim 17, wherein the TF-related disease is selected from the group consisting of: a cancer, thrombosis, autoimmune disease, metabolic-related disease, infectious disease, and a combination thereof; preferably selected from the group consisting of: breast cancer, lung cancer, pancreatic cancer, ovarian cancer, prostate cancer, colorectal cancer, glioma, melanoma, leukemia, lymphoma, and a combination thereof.
